(19) Europäisches Patentamt
European Patent Office
Office européen des brevets

(11) Publication number: **0 181 635**
**A2**

# (12) EUROPEAN PATENT APPLICATION

(21) Application number: 85114436.0

(22) Date of filing: 13.11.85

(51) Int. Cl.⁴: C 07 H 21/00
G 01 N 33/53, C 07 K 15/00
C 12 Q 1/68, A 61 K 39/395
//G01N33/577

(30) Priority: 14.11.84 US 671296
26.06.85 US 749178

(43) Date of publication of application:
21.05.86 Bulletin 86/21

(84) Designated Contracting States:
AT BE CH DE FR GB IT LI LU NL SE

(71) Applicant: Oncogene Science, Inc.
222 Station Plaza North
Mineola New York 11501(US)

(72) Inventor: Groffen, John
46 Kenilworth Road
Mineola New York 11501(US)

(72) Inventor: Heisterkamp, Nora
46 Kenilworth Road
Mineola New York 11501(US)

(72) Inventor: Stephenson, John R.
74 Strathmore Lane
Rockville Center New York, 11570(US)

(74) Representative: Henkel, Feiler, Hänzel & Partner
Möhlstrasse 37
D-8000 München 80(DE)

## (54) Probes and methods useful for detecting chromosomal translocations.

(57) A single-stranded nucleic acid molecule useful as a probe for detecting chromosomal translocations has a nucleotide sequence which comprises at least one sequence present within an exon of a eucaryotic chromosomal gene and complementary to the mRNA encoded by the exon, the gene being characterized by the presence within it of at least one chromosomal breakpoint site into which another nucleotide sequence may be translocated. In a specific embodiment the translocation is the Philadelphia translocation, the eucaryotic chromosomal gene is on human chromosome 22 and the translocated nucleotide sequence is at least part of the *c-abl* oncogene.

The invention also provides purified, hybrid mRNA molecules useful as indicators of chromosomal translocations. The mRNAs are transcripts of hybrid DNA sequences present on aberrant chromosomes resulting from chromosomal translocations. The invention further provides hybrid polypeptides encoded by the hybrid mRNA molecules and antibodies directed to antigenic determinants on these hybrid polypeptides.

The single-stranded nucleic acid molecules, polypeptides and antibodies of this invention may be used to detect chromosomal translocations and to diagnose diseases associated therewith.

EP 0 181 635 A2

Croydon Printing Company Ltd

# PROBES AND METHODS USEFUL FOR DETECTING CHROMOSOMAL TRANSLOCATIONS

This application is a continuation-in-part of U.S. Serial No. 671,296, filed November 14, 1984, the contents of which are hereby incorporated by reference into this application.

## Background of the Invention

Throughout this application various publications are referenced by numbers within parentheses. Full citations for these publications may be found at the end of the specification immediately preceding the claims. The disclosures of these publications in their entireties are hereby incorporated by reference into this application in order to more fully describe the state of the art to which this invention pertains.

Certain specific chromosomal translocations are known to be associated with human cancer. The chromosomal rearrangements that occur as a result of these translocations may activate human cellular oncogenes that have been rearranged.

Chronic myelocytic leukemia (CML) is a pluripotent stem cell disease characterized by the presence of the Philadelphia (Ph') chromosome in the leukemic cells of 96% of all CML patients. The Ph' chromosome is the result of a translocation between chromosomes 22 and 9 (1). The human c-abl oncogene has been mapped to the long (q) arm of chromosome 9 (2). By analysis of somatic cell hybrids, we have shown that this oncogene is located on the Ph' ($22q^-$) chromosome in Ph' positive CML demonstrating that c-abl is involved in the 9; 22 translocation (3). The location of the c-abl oncogene adjacent to the translocation breakpoint in CML was shown by the isolation of a DNA fragment from the $9q^+$ chromosome of a CML patient: this fragment contained both sequences of chromosome 9 and 22. The breakpoint had occurred 14 kb immediately 5' of the v-abl homologous sequences and resulted in a $9q^+$ chromosome in which the tip of chromosome 9 including the v-abl homologous sequences was replaced by a portion of chromosome 22 (4). The isolated chromosome 22 sequences of this hybrid DNA fragment enabled us to study their role in the Ph' translocation in greater detail. A breakpoint cluster region (bcr) was identified on chromosome 22; the DNAs of all (over 30) Ph' positive CML patients examined to date have breakpoints in this region of up to 5.8 kb, strongly suggesting a role for bcr in chronic myelocytic leukemia (5). Although patients with the Ph' translocation could be identified using bcr specific genomic probes, see our pending patent application U.S. Serial No. 571,911 filed January 18, 1984, the contents of which are hereby incorporated by reference,this was experimentally difficult and in many cases it involved the use of several distinct probes.

In 1975, Lozzio and Lozzio (6) reported the isolation of a cell line K-562, from the pleural fluid of an adult patient with CML. This cell line expresses phenotypic markers of erythroid lineage and displays induced and spontaneous globin synthesis (7). We and others (4, 8, 9) have shown that the c-abl oncogene and the immunoglobulin light chain constant region (C) are amplified at least fourfold in this cell line. In contrast, another human oncogene, c-sis, normally located on chromosome 22 but transposed to chromosome 9 in the Ph' translocation (10), is non-amplified (4). These data suggest that K-562 contains a Ph' chromosome which is at least fourfold amplified; cytogenetic data are, however, not confirmative, as we cannot detect a Ph' chromosome in different pedigrees of this cell line and others (6) have suggested the presence of a single Ph' chromosome. Such findings leave the question as to whether amplification of the c-abl oncogene in K-562 is correlated with the presence of an amplified Ph' translocation, unresolved. If such a correlation exists, it would unambiguously demonstrate that K-562 is derived from Ph' positive leukemic cells of a CML patient. We demonstrate the presence of a Ph' chromosomal breakpoint in the DNA of K-562. The breakpoint has occurred in bcr confirming our previous results that a breakpoint in bcr is highly specific for CML. These findings establish that the progenitor cell of K-562 contains the Ph' translocation and that C and c-abl are located on the same amplification unit.

The present findings demonstrate that the CML cell line K-562, as all Ph' positive CML patient material examined to date, contains a breakpoint on chromosome 22 within the breakpoint cluster region on chromosome 22. However, K-562 DNA contains in contrast to DNAs isolated from patient material, amplified remnants of the Ph'-chromosome, including C, a part of bcr and c-abl. These amplified regions do not represent multiple copies of intact Ph'-chromosomes, but rather are present on one acrocentric marker chromosome (9). Most probably, the regions originate from a multiplication of a large region of DNA from the original Ph' chromosome. Restriction enzyme analysis with various restriction enzymes shows that all copies of the bcr breakpoint region contain identically sized breakpoint fragments.

Although no difference in the degree of c-abl amplification can be observed in different passages of K-562, there is no evidence available that the original leukemic cells of the patient from which K-562 was established contained more than one Ph'-chromosome. More likely, amplification occurred after establishment of the cell line, leading to selection of cells with a growth advantage. K-562 cells also differ from other CML cells in that the $9q^+$ chromosome cannot be detected by Southern blot analysis. This in concordance with results of cytogenetical analysis in which the $9q^+$ chromosome was found to be absent from K-562 (9).

The absence of the $9q^+$ chromosome strengthens the hypothesis that the $22q^-$ chromosome is critical to the malignant proliferation of these leukemic cells. In K-562, the amplification of human c-abl DNA sequences is coupled to an increased level of expression of an abnormally sized 8.5 kb c-abl mRNA, (12, 19) indicating active transcription from the translocated and amplified c-abl oncogene. Recently, an altered human c-abl protein was demonstrated in K-562, differing both in size and in the presence of associated protein kinase activity from the normal human protein (21). However, these previous studies of altered c-abl protein and mRNA did not determine that these molecules were hybrid molecules. We demonstrate that in fact a hybrid mRNA and protein product exist as a result of the Philadelphia translocation. We have invented novel diagnostic tests for CML and other diseases associated with similar translocations based on the hybrid nature of these aberrant gene products.

This invention concerns construction and use of DNA probes to detect aberrant chromosomes resulting from translocations such as the Philadelphia translocation. The invention also concerns methods of detecting these same translocations by assaying for mRNA and protein products derived from these aberrant chromosomes.

Summary of the Invention

A single-stranded nucleic acid molecule is provided that is useful as a probe for detecting chromosomal translocations and has a nucleotide sequence which comprises at least one sequence present within an exon of a eucaryotic chromosomal gene and complementary to the mRNA encoded by the exon. The eucaryotic chromosomal gene is characterized by the presence within it of at least one chromosomal breakpoint site into which another nucleotide sequence may be translocated.

In one embodiment of the invention the sequences are present within exons located 3' of chromosomal breakpoint sites into which an oncogene may be translocated. In a presently preferred embodiment of the invention, the translocation is the Philadelphia translocation, the eucaryotic chromosomal gene is on human chromosome 22 and is characterized by the presence within it of a breakpoint cluster region and the translocated sequence is at least part of the c-abl oncogene.

The single-stranded nucleic acid molecules may be DNA or RNA, preferably from about 20 to about 8,500 base pairs in length and may be labelled with detectable moieties, e.g. radioactive isotopes. These labelled probe molecules may used to detect chromosomal translocations, e.g. the Philadelphia translocation.

The invention also provides a purified, mRNA molecule useful as an indicator of a chromosomal translocation. The mRNA is a transcript of a DNA sequence which comprises at least a portion of an exon of a eucaryotic chromosomal gene and at least a portion of another nucleotide sequence which has translocated into the gene. In one embodiment of the invention the nucleotide sequences are present within exons located 5' of the translocated sequence. In a presently preferred embodiment invention the nucleotide sequences are located on the breakpoint cluster region of human chromosome 22 (bcr) and the translocated nucleotide sequence is at least part of the c-abl oncogene.

The invention further provides polypeptides encoded by the purified, mRNA molecules, e.g. a bcr/c-abl polypeptide encoded by the bcr/c-abl mRNA molecule. Antibodies, e.g. monoclonal antibodies, may be produced which are directed to antigenic determinants on this polypeptide. These antibodies may be used to detect a polypeptide, e.g. a bcr/c-abl polypeptide, present as a result of a chromosomal translocation, e.g. the Philadelphia translocation, and to diagnose an associated disease, e.g. chronic myelocytic leukemia, acute lymphocytic leukemia or acute myelocytic leukemia.

Brief Description of the Figures.

Fig. 1. Restriction enzyme map of the K-562 breakpoint region on chromosome 22.

A is the restriction enzyme map of human chromosome 22 sequences: the 5.8 kb Bgl II - Bam HI (bcr) region encompasses the 0.6 HB and 1.2 HBg probes. D is the restriction enzyme map of the Ph' chromosome in K-562: the Ph' chromosomal breakpoint is indicated with an arrow. B and C represent more detailed restriction enzyme maps of the indicated regions of A and D. The solid bars represent chromosome 22 sequences whereas the open bars indicate sequences originating from chromosome 9. Probes used in the study are shown above A and below D. (A= Ava I; B= Bam HI; Bg= Bgl II; E= Eco RI; H= Hind III; K= Kpn I; P= Pst I; S= Sst I; Xh= Xho I.)

Fig. 2. Restriction enzyme map of the plasmid VI-3 cDNA insert.

The isolation of the VI-3 cDNA plasmid is described in detail in the specification. Poly G represents the 5' end of the cDNA; the poly A tail of the 3' is also indicated. The breakpoint in K562 is in an intron; exons 5' of the arrow will remain on chromosome 22 and constitute part of the hybrid mRNA. Probes A and B are 5' and 3' fragments of the cDNA subcloned into a pSP vector. (Poly A tail= 3'; Pv= Pvu II; Bg= Bgl II; H= Hind III; Ps= Pst I; A= Ava I.)

Fig. 3. The DNA sequence and translation of VI-3 sequence.

Amino acid residues are indicated with one letter symbol below the sequence. The exact position of exons designated 1-6 within VI-3 bcr cDNA is shown by the numbers above the sequence. DNA sequencing was performed using the dideoxy-chain termination method on restriction enzyme fragments of the cDNA subcloned into M13 phage. All regions were sequenced on both strands.

Fig. 4. bcr cDNA VI-3.

A detailed restriction enzyme map of VI-3 is shown. The black bar indicates the open reading frame sequences, the thin line non-translated sequences. The 3' end of the cDNA containing the poly A tail is depicted as $A_n$. (A = Ava I, Ap = Apa I, B = BstE II, H = Hind III, Hf = Hinf I, P = Pst I, PII = Pvu II, S = Sau 3A, Bg = Bgl II, Bs = Bst E II.)

Fig. 5. DNA sequence analysis of the K-562 breakpoint.

Top: normal chromosome 22 DNA sequences. Bottom: the corresponding DNA sequences of the breakpoint region in K-562. An 0.37 kb Ava I/Eco RI fragment of K-562 (Fig. 1C) and an 0.37 kb Ava I/Pst 1 (Fig. 1B) fragment of normal chromosome 22 were sequenced according to the dideoxy method essentially as described in 22. The region shown is located 241 bp 3' to the Ava I site.

Fig. 6. Schematic representation of the molecular consequence of the Ph translocation.

In the Ph -translocation, a break occurs within an intron of the bcr gene on chromosome 22 and within or 5' to the c-abl oncogene on chromosome 9; these DNA sequences are physically joined on the Ph chromosome in a head to tail fashion; with bcr centromeric and c-abl telomeric. This configuration allows for the transcription of a hybrid mRNA, consisting of 5' bcr exons fused to c-abl coding sequences. This mRNA is translated into a hybrid polypeptide with a bcr amino-terminus and c-abl carboxy-terminus.

Fig. 7. Genomic organization of the bcr gene.

The restriction enzyme map of chromosome 22 sequences encompassing bcr is shown in A. Exons are indicated by black boxes below the restriction enzyme map. The position of the numbered exons were located by hybridization to the VI-3 cDNA. The asterisk indicates a polymorphic Bgl II restriction enzyme site. A restriction enzyme map of the breakpoint cluster region is shown in B, with the exons as indicated in A. Below the map, the approximate positions of the breakpoints in different CML DNAs are indicated by horizontal or vertical arrows. (A = Ava I, B = BamHI, Bg = Bgl II, E = EcoRI, H = Hind III, P = Pst I, S = Sma I, Ss = Sst I, X = Xho I.)

The breakpoint of the $22q^-$ chromosome from DNA 0319129 was cloned as a 9.5 kb Bgl II fragment in Charon 30 according to previously described methods (37). 9q+ fragments were isolated by cloning a 7.2 kb Bam HI fragment and a 7.7 kb EcoRI fragment into charon 30 and lambda gt wes respectively.

Fig. 8. Breakpoint sequences of the DNAs of 2 CML patients.

The sequence of 0319129 DNA is shown in A; the sequences are in a 5' - 3' orientation. Normal chromosome 9 sequences (first line) are from non-CML DNA; the 9q+ and 22q− sequences (second and third lines) from DNA of the patients 0319129. Normal chromosome 22 sequences (fourth line) are from non-CML DNA. An arrow points to the breakpoint on chromosomes 9 and 22; the nucleotide C found both in the 9q+ and 22q− sequences at the breakpoint is boxed. Limited regions of homology between the normal chromosome 9 and 22 sequences are underlined. In B, the breakpoint sequence of 02120185 DNA is shown; normal chromosome 9 and 22 sequences (first and third line in each set) were from non-CML DNA. The 9q+ sequence on the second line contains an area boxed to indicate it does not originate from the normal chromosome 9 or 22 sequenced in the present experiments. Dots above the chromosome 9 sequences indicate nucleotide differences at those positions with the 9q+ chromosome. The beginning of exon 3 (see Fig. 6B) in the 9q+ and 22 sequence is indicated in the figure. Small restriction enzyme fragments containing the breakpoints were chosen for sequence analysis, based on restriction enzyme mapping data and comparison with normal chromosome 9 and 22 maps.

Fig. 9. Exon-intron organization with bcr.

A. Restriction enzyme map of bcr; the 5.8 kb breakpoint cluster region, in which all breakpoints occur on chromosome 22 in the Ph'-translocation, is indicated above the map. The location of exons immediately 5', 3' and within bcr are schematically shown (not drawn to scale) beneath the map. Number exons are referred to in text.

B. Restriction enzyme map of the bcr cDNA; numbers and shading of regions in the cDNA correspond to the exons similarly marked in A. The bcr cDNA probes used in this study are shown beneath the map. (A = Ava I, B = BamH I, Bg = Bgl II, Bs = BstE II, E = EcoRI, H = Hind III, P = Pst I, Pv = Pvu II. The $AAA_N$ at the 3' end of the cDNA indicates the poly A tail.)

Detailed Description of the Invention

The present invention concerns a single-stranded nucleic acid molecule useful as a probe for detecting a chromosomal translocation. The molecule has a sequence which comprises at least one sequence present within an exon of a eucaryotic chromosomal gene and complementary to the mRNA encoded by the exon. The eucaryotic chromosomal gene is characterized by at least one chromosomal breakpoint site into which another nucleotide sequence may be translocated.

The invention also concerns nucleic acid molecules which are substantially complementary to these single-stranded nucleic acid molecules. These complementary nucleic acid molecules may also be used as probes for nucleic acid hybridization analysis of chromosomal breakpoints.

The single-stranded nucleic acid molecule may be a DNA molecule or an RNA molecule and may be comprised exclusively or essentially of sequences present within exons or alternatively may be comprised of sequences present within both exons and introns. Desirably, at least one sequence present within an exon is located 3' of the chromosomal breakpoint site or sites. Typically, a plurality of sequences present within a plurality of exons are employed and are located 3' of the chromosomal breakpoint sites.

This single-stranded nucleic acid molecule is of a length that permits the use of the molecule as a probe in nucleic acid hybridization experiments, preferably from about 20 base pairs to about 8,500 base pairs. DNA molecules of different sizes may be prepared by methods known to those of ordinary skill in the art, e.g. restriction enzyme cleavage or oligonucleotide synthesis.

Preferably, the eucaryotic chromosomal gene in which the exon nucleotide sequences are located is a human chromosomal and is characterized by the presence within it of a breakpoint cluster revision (bcr). The breakpoint site may be located within an intron or an exon. In one embodiment of this invention, the gene is located on human chromosome 22 and the exon nucleotide sequences correspond to one or more of the bcr family of nucleotide sequences. In a specific embodiment of the invention, the gene is located in the bcr which is specifically involved in the Philadelphia translocation in patients with chronic myelocytic leukemia (CML). In over 90% of patients with CML and a lower, but significant, fraction of patients with acute myelocytic leukemia (AML) and acute lymphocytic leukemia (ALL), at least part of the nucleotide sequences of the c-abl oncogene which is normally located on chromosome 9 are translocated to chromosome 22 in the Philadelphia translocation. The construction of a single-stranded DNA molecule of this embodiment of the invention is described in the Experimental Section of the specification. Several different probe molecules may be constructed from this DNA molecule by methods such as restriction enzyme cleavage or oligonucleotide synthesis. For instance, the plasmid VI-3 may be used to construct several different probes as shown in Fig. 2. This molecule has been sequenced and this sequence appears in the Fig. 3. Other single-stranded DNA molecules useful as probes for other chromosomal translocations may be constructed using the methods of this invention as described in the Experimental Section.

The single-stranded DNA molecules of this invention may be included in a double-stranded DNA molecule, thus vector molecules may be constructed which comprise the DNA molecules of this invention, i.e. the DNA molecule may be incorporated as an insert in a vector such as a plasmid, e.g. the plasmid VI-3, a cosmid or a bacteriophage. These vector molecules may be prepared by standard recombinant DNA techniques known to those of ordinary skill in the art. Alternatively, the molecules may be subcloned into plasmid vectors, such as the SP6 vector series, (26, 27) for the generation of complementary RNA molecules suitable for use as probes in hybridization procedures.

The single-stranded DNA molecules of this invention may be labelled with detectable moieties. These labelled molecules may then be used in various nucleic acid hybridization methods known to those of ordinary skill in the art. The molecules may be labelled with any type of a detectable moiety such as a fluorescent, enzymatic or radioactive marker, e.g. by nick translation with $^{32}P$.

These labelled molecules may be used to detect chromosomal translocations on a given eucaryotic chromosome. One such method comprises cleaving the chromosomal DNA from the chromosome with an appropriate restriction enzyme or enzymes under suitable conditions so as to produce DNA fragments. The resulting fragments are treated to obtain single-stranded DNA molecules. These single-stranded molecules are contacted with labelled single-stranded nucleic acid probe molecules of this invention under suitable conditions permitting hybridization of complementary molecules. The presence of hybridized molecules is detected and thereby the presence of a chromosomal translocation is detected by analyzing the results of the hybridization.

The invention further provides a method of detecting a chromosomal translocation which comprises cleaving the chromosomal DNA with an appropriate restriction enzyme or enzymes and treating the resulting fragments by gel electrophoresis and denaturation to obtain single-stranded DNA molecules. The single-stranded DNA molecules are recovered from the gel and immobilized on a suitable solid support, e.g. a nitrocellulose filter. The immobilized single-stranded DNA fragments are then contacted with the labelled single-stranded nucleic acid probe molecules of this invention under suitable conditions permitting hybridization of complementary single-stranded molecules. The hybridized molecules so formed are identified, e.g. by autoradiography, and abnormalities in the restriction patterns of the chromosomal DNA caused by the chromosomal translocation are identified.

A specific embodiment of the invention concerns a method of detecting the Philadelphia translocation in a human subject which comprises cleaving the subject's total chromosomal DNA with an appropriate restriction enzyme or enzymes under suitable conditions so as to produce DNA fragments. The resulting fragments are then treated to obtain single-stranded DNA molecules. The single-stranded DNA molecules so obtained are contacted with single-stranded nucleic acid molecules complementary to exon nucleotides of the bcr region of chromosome 22 under suitable conditions permitting hybridization of complementary molecules. The presence of the hybridized molecules is detected and thereby the chromosomal translocation is detected.

One such method concerns detecting the Philadelphia translocation in a human subject by cleaving the subject's total chromosomal DNA with an appropriate restriction enzyme or enzymes under suitable conditions so as to produce DNA fragments. The resulting fragments so obtained are treated by gel electrophoresis and denaturation to obtain single-stranded DNA molecules. The single-stranded DNA molecules so obtained are recovered from the gel and immobilized on a suitable solid support, e.g. a nitrocellulose filter. The immobilized, single-stranded DNA molecules are then contacted with the labelled, single-stranded nucleic acid molecules complementary to the exons of the _bcr_ region of chromosome 22, e.g. a fragment of an insert of the plasmid VI-3 such as probe A, Fig. 2, under suitable conditions permitting hybridization of complementary single-stranded molecules. The hybridized molecules so formed are identified e.g. by autoradiography, thereby detecting abnormalities in the restriction patterns of the chromosomal DNA caused by the Philadelphia translocation.

The methods of using the probe molecules of this invention are not limited to those methods discussed herein. The probes of this invention may also be used in other nucleic acid hybridization methods known to those of ordinary skill in the art, e.g. _in situ_ hybridization.

The present invention also concerns a purified, hybrid mRNA molecule useful as an indicator of a chromosomal translocation. The mRNA molecule is a transcript of a DNA sequence which comprises at least a portion of an exon nucleotide sequence of a eucaryotic chromosomal gene and at least a portion of another nucleotide sequence which has translocated into the gene.

In certain embodiments of the invention the exon nucleotide sequences of the gene are located 5' of the translocated sequence. In other embodiments the translocated nucleotide sequence is at least part of an oncogene. In another embodiment of the invention the exon nucleotide sequences 5' of the translocated sequence are derived from either the bcr region of chromosome 22 or from one or more of the three bcr related sequences on chromosome 22. In one specific embodiment the purified mRNA molecule is a bcr/c-abl mRNA molecule from about 6.0 to about 9.0 kb in length. This molecule is a result of the Philadelphia translocation in which the c-abl oncogene from chromosome 9 is translocated to chromosome 22. The identification and isolation of this molecule is described in the experimental section of the specification.

The mRNA molecules of this invention may be purified by any means known to those of ordinary skill in the art, e.g. purified by gel electrophoresis, column chromatography, density gradient centrifugation or nucleic acid hybridization methods. In certain embodiments they may be isolated and purified by hybridizing them to single-stranded DNA molecules which are substantially complementary to the mRNA molecule that is to be purified. Such single-stranded c-DNA molecules may be bound to a solid support, e.g. a c-DNA molecule complementary to the bcr/c-abl mRNA bound to cellulose beads or a nitrocellulose filter. After hybridization of complimentary strands unbound nucleic molecules may be washed off. The nucleic acid duplexes may then be denatured and the mRNA molecules may be obtained in a purified state.

The purified, hybrid mRNA molecules of this invention may also be used to prepare DNA or cRNA probes. Once the mRNA molecules have been purified, cDNA or cRNA can be produced enzymatically, e.g. cDNA produced with reverse transcriptase. These cDNA molecules may then be cloned in a suitable vector system by standard recombinant DNA methods known to those of ordinary skill in the art. Various probe molecules may then be constructed from the cloned cDNA. Alternatively the purified mRNA or cRNA may be labelled with a detectable moiety and used as a probe, e.g. enzymatically labelling the RNA with $^{32}P$ by polynucleotide kinase. This mRNA probe may then be used to identify and isolate the translocated genes present on the aberrant chromosome by using standard molecular biology techniques known to those of ordinary skill in the art.

The current invention also concerns polypeptides encoded by the purified, hybrid mRNA molecules. These polypeptides contain polypeptide sequences which are encoded for originally by two different genes. One such polypeptide is the bcr/c-abl protein encoded for by the bcr/c-abl mRNA. This polypeptide may contain a portion encoded for by the exon nucleotide sequences of the bcr region of chromosome 22 and a portion encoded by the oncogene c-abl.

Antibodies to these polypeptides may also be prepared which may be directed against antigenic determinants on the polypeptide, e.g. determinants specifically created by the fusion of the two genes, e.g. determinants present in neither of the proteins of the fused genes separately, but present in the hybrid protein. Such antibodies may be used in various diagnostic or therapeutic methods. In other embodiments of the invention more than one antibody to the polypeptide may be prepared, each antibody directed to an antigenic determinant present on a different portion of the polypeptide. One such antibody may be directed to an antigenic determinant present on the portion of the polypeptide encoded for by the gene located on the chromosome into which another sequence has translocated, e.g. the exon nucleotide sequences on chromosome 22 encoding the bcr region of the bcr/c-abl polypeptide. The other antibody may be directed to an antigenic determinant located on the portion of the polypeptide encoded for by the nucleotide sequence that has been translocated into the chromosome, e.g. a portion encoded for by an oncogene such as the oncogene c-abl. These antibodies may be used to diagnose a disease related to a chromosomal translocation which produces a specific protein product, e.g. CML evidenced by the Philadelphia translocation and the presence of the bcr/c-abl polypeptide. In certain embodiments of the invention two different antibodies directed to two different sites on the polypeptide may be used in immunometric or sandwich type assays.

In a preferred embodiment of the invention, either or both of the antibodies are monoclonal antibodies. The antibodies of this invention may also be labelled with detectable moieties, such as radioactive, e.g. $^{125}I$, fluorescent, e.g. fluorescein, or enzymatic, e.g. peroxidase, moieties.

Suitable conditions for formation of an antibody-antigen complex comprise solution phase and emulsion reaction systems, preferably aqueous, maintained at a pH value from about 6.0 to about 8.0 and at a temperature from about 4°C to about 45°C.

The polyclonal antibodies and monoclonal antibodies of this invention may be prepared by methods known to those of ordinary skill in the art. The monoclonal antibodies may be prepared by techniques as disclosed by Kohler, et al., Nature Vol. 256: 995-497 (1975). The process involves injecting a mouse or other animal with an immunogen. The mouse is later sacrificed and cells taken from its spleen are fused with myeloma cells, producing hybridomas. The population of hybridomas produced in vitro is screened to isolate individual clones each of which secretes a single antibody species to the antigen. The antibodies produced by the hybridomas are then screened to identify those having the highest affinity for the immunogenic substance of interest.

Monoclonal antibodies directed to different portions of a hybrid polypeptide may be produced by using the entire polypeptide itself as an immunogen and then screening the hybridomas produced for antibodies directed to antigenic determinants on different portions of the polypeptide. Alternatively, fragments of the various portions of the hybrid polypeptides, e.g. a portion of the bcr/c-abl polypeptide which is encoded by the exon nucleotide sequences present on the bcr region of chromosome 22, may be used as immunogens. For either purpose, large amounts of the polypeptide could be produced with knowledge of the restriction data disclosed herein and use of standard prokaryotic and eucaryotic expression systems known to those of ordinary skill in the art. Another approach is to use synthetic peptides analogous to portions of the hybrid polypeptide as an immunogen, e.g. a synthetic polypeptide analogous to an antigenic determinant present on the bcr portion of the bcr/c-abl polypeptide.

One embodiment of the invention concerns a method of detecting a chromosomal translocation, e.g. the Philadelphia translocation by determining the presence of a polypeptide encoded by a mRNA resulting from the translocation, e.g. the bcr/c-abl polypeptide. The method comprises contacting a sample containing the polypeptide with an antibody directed to an antigenic site on the polypeptide, the antibody being labelled with a detectable moiety, under suitable conditions permitting the formation of an antibody-antigen complex. The antibody may be a polyclonal or monoclonal antibody. The unbound, labelled antibody is then separated from the labelled antibody-antigen complex, and the presence of the labelled antibody-antigen complex is then detected by suitable means, e.g. autoradiography or fluorescent detection.

The antibodies of this invention, both polyclonal and monoclonal, may also be used in a method of detecting chromosomal translocations in which two different antibodies directed against antigenic determinants on different fractions of the polypeptide are used. In this embodiment, a sample containing the polypeptide is contacted with a measured amount of a first antibody directed to an antigenic site on the portion of the polypeptide encoded for by the exon nucleotide sequences of the gene into which another nucleotide sequence has been translocated under suitable conditions so that an antibody-antigen complex is formed. The first antibody is labelled with a detectable moiety. The labelled antibody-antigen complex is then contacted with a second antibody directed to an antigenic site

on the portion of the polypeptide encoded for by the nucleotide sequence which has translocated into chromosome, e.g. an oncogene, under suitable conditions permitting the formation of a labelled antibody-antigen-antibody complex. The second antibody is bound to a suitable solid support, e.g. a microtiter well, a cellulose bead, a nitrocellulose filter. The unbound labelled antibody is then separated from the labelled antibody-antigen-antibody complex bound to the solid support and then either the amount of bound labelled antibody-antigen-antibody complex or the amount of unreacted labelled antibody is measured in order to determine the presence of the polypeptide.

Alternatively this procedure may be conducted by using a method wherein the first antibody is not labelled but is attached to the solid support and the second antibody is labelled with the detectable moiety.

A specific embodiment of the invention concerns a method of detecting the Philadelphia translocation by detecting the presence of the bcr/c-abl polypeptide. The method comprises contacting a sample obtained from the subject with a measured amount of first antibody to an antigenic site on the portion of the polypeptide encoded for by the c-abl oncogene, under suitable conditions permitting the formation of an antibody-antigen complex. The first antibody is labelled with a detectable moiety. The complex so formed is contacted with a second antibody to an antigenic site on the portion of the polypeptide encoded for by exon nucleotide sequences of the gene on chromosome 22, under suitable conditions permitting the formation of a labelled antibody-antigen-antibody complex. The second antibody is bound to a suitable solid support. The unbound labelled antibody is separated from the labelled antibody-antigen-antibody complex bound to the solid support. Either the amount of labelled antibody-antigen-antibody complex bound to the solid support or the unbound labelled antibody is measured in order to detect the presence of the polypeptide. Alternatively, this procedure may be conducted by using a method wherein the first antibody is not labelled but is attached to the solid support and the second antibody is labelled with a detectable moiety.

Another method of detecting a chromosomal translocation on a given eucaryotic chromosome such as the Philadelphia translocation comprises isolating poly A mRNA molecules encoded for by genes on the chromosome, e.g. mRNA from a human subject which contains mRNA encoded by genes on the Ph chromosome, and separating the poly A mRNAs, e.g. by gel electrophoresis. The separated RNA molecules are immobilized on a suitable solid support, e.g. immobilized in the dried gel or on a nitrocellulose filter, and contacted with labeled probe RNA molecules of this invention under suitable conditions permitting hybridization of complementary molecules. The presence of the hybridized molecules is detected and thereby the presence of the chromosomal translocation is detected.

Another embodiment of the invention concerns methods of diagnosing CML, AML or ALL in a human subject by detecting the presence of the Philadelphia translocation.

Experimental Details

In DNAs of the majority of Ph' positive CML patients examined to date, the presence of a breakpoint on chromosome 22 can be demonstrated using a 1.2 HBg probe (5 and Fig. 1A). For example, abnormal EcoRI restriction enzyme fragments were clearly present in the DNAs of a representative group of CML patients designated 02120185, 0319129 and 0311068. Restriction enzyme fragments containing these breakpoints have been molecularly cloned (4, 5) and shown to represent 9q+ fragments. In K-562, a cell line isolated from the pleural fluid of an adult with CML, abnormal DNA fragments could not be detected either with EcoRI or with each of several other restriction enzymes tested after hybridization to the 1.2 HBg probe. Similarly, DNAs from several additional Ph' positive CML patients could not be shown as Ph' positive by use of this probe. This could indicate that certain Ph' positive leukemias, including K-562, do not contain breakpoints on chromosome 22 within bcr or alternatively, that use of genomic DNA probes, such as 1.2 HBg is inadequate for their detection.

To examine this more thoroughly, a probe more to the 5' (0.6 HB, see Fig. 1A) within bcr was prepared, and hybridized to the DNA of K-562 digested with different enzymes. (5) This probe detects, in addition to the normal 5.0 kb Bgl II fragment, abnormal Bgl II fragments in K-562 DNA. Moreover, one of these fragments is amplified at least fourfold. Abnormal amplified restriction enzyme fragments in K-562 could also be detected using other enzymes. Since the 0.6 HB probe has detected 22q⁻ fragments in the DNAs of a number of CML patients (5), it seemed likely that the abnormal amplified fragments in K-562 represent amplified sequences on the 22q⁻ chromosome.

To analyze this in more detail, a cosmid library was constructed from K-562 DNA partially digested with Mbo I, according to published procedures (11). Numerous colonies of the approximately 100,000 recombinants hybridized with 0.6 HB probe. Three such positives containing overlapping portions of the same region were selected for further restriction enzyme analysis (Fig. 1D). It is evident from a comparison of the detailed restriction enzyme maps of normal chromosome 22 sequences (Fig. 1B) and K-562 DNA (Fig. 1C) that the homology between the two terminates 3'to the most 5' Ava I site.

A 1.0E probe prepared from K-562 DNA immediately 3' to the breakpoint (see Fig. 1D) hybridizes to DNA isolated from somatic cell hybrids containing human chromosome 9 in the absence of human chromosome 22, but not from hybrids containing chromosome 22. This indicates that the sequences isolated from K-562 DNA come from different chromosomes and contain the breakpoint of the $22q^-$ chromosome. The entire region is amplified at least fourfold. The chromosome 9 specific sequences are also amplified, as demonstrated by the strong hybridization of the 1.0E probe to K-562 DNA in comparison with control DNA. Thus the amplification of chromosome 9 sequences begins at the point where the breakpoint has occurred on chromosome 9 in the Ph' translocation and extends in the direction of the telomere of the chromosome, including the c-abl oncogene. The amplified region may be relatively large, as the distance between the breakpoint on chromosome 9 and the most 5' v-abl homologous exon is, at minimum, 64 kb.

These results indicate that the cell line K-562 does contain a breakpoint within bcr; however, this breakpoint could not be detected with a probe originating from the 3' region of the *bcr*. To obtain probes which can detect the Ph' breakpoint in all patient material and to examine if the *bcr* region is part of a gene, experiments were initiated to isolate a *bcr*-specific cDNA. Of several genomic DNA probes tested, the 0.6HB probe (Fig. 1) was found to hybridize to RNA on a Northern blot, indicating that it contains one or more exons. The 0.6 HB probe therefore, was used to screen a human cDNA library.

Ten micrograms of GM 637 cDNA containing plasmid were obtained from Hiroto Okayama. This library is a cDNA library of the SV-40 transformed human fibroblast cell line GM 637. The construction of the library is described in Okayama and Berg (21). It would be clear to one skilled in the art that any representative human cDNA library may be used. It is also obvious that one skilled in the art could prepare a comparable cDNA library from the human GM 637 cell line, which is available from the NIGMS Human Genetic Mutants Cell Repository, Camden, New Jersey.

*E. coli* DH1 was transformed with the 10 micrograms of GM 637 cDNA containing plasmids by standard recombinant DNA procedures known to those of ordinary skill in the art. The transformed *E. coli* cultures were plated out on ten large plates. Replicas of these plates were made and the bacteria were scraped off for culturing. An SV-cDNA library was constructed by preparing ten large scale plasmid preparations from these recombinant bacteria. The preparations were designated SV-1 thru SV-10.

Ten micrograms of each plasmid preparation was digested in 100 microliters of SalI restriction enzyme buffer with 42 units of the restriction enzyme SalI. The digestion was for 4 hours under suitable buffer and temperature conditions known to those of ordinary skill in the art. After digestion each of the DNA samples was extracted with phenol and ethanol precipitated. The samples were then electrophoresed on a 0.6% agarose gel for 4 days at 5-18 volts. The DNA in the gel was transferred to a nitrocellulose filter (Schleicher & Schuell) according to the procedure of Southern. (15).

The 0.6 HB probe was nick translated with $^{32}P$ and was hybridized to the cDNA samples immobilized on the nitrocellulose filters according to procedures known to those of ordinary skill in the art. (17). After hybridization was completed the filters were washed under the high stringency conditions of 0.3 x SSC at 65°C. Autoradiography of the washed filters showed hybridization to DNA bands of about 5 kb in all of the samples except the sample of preparation SV-6.

The entire SV-cDNA library was then replated onto large nitrocellulose filters (Millipore HATF). Replicas of these filters were made and hybridized to the 0.6 HB probe. Positive colonies were replated onto small nitrocellulose filters at a density suitable for the isolation of single colonies. These filters were replicated and hybridized to the 0.6 HB probe. Colonies that hybridized to the probe were picked and grown up on a scale suitable for large scale DNA isolation.

Two of the positive colonies consisting of the plasmids VI-3 and VII-1 were further analyzed. Of these, the plasmid VI-3 contained the largest insert, about 2.2 kb, and was chosen for still further analysis. A detailed restriction enzyme map of this insert was constructed (Fig. 2) and the 5'-3' orientation of the cDNA was determined.

The cDNA insert of plasmid VI-3 was then used as a probe in hybridization experiments with Southern blots of genomic DNA of human chromosome 22 including the bcr region. This region of chromosome 22 is contained in the cosmid clones Ca 22-1 and Ca 22-2. The cosmid clone preparation, restriction digest, electrophoresis, blotting and hybridization procedures were performed according to standard methods known to those of ordinary skill in the art. Numerous restriction enzyme fragments which are not contiguous in the genomic DNA hybridized to the VI-3 probe indicating that this cDNA is indeed a reverse transcribed copy of part of an mRNA encoded for by a gene that encompasses within its coding region the bcr.

The VI-3 cDNA insert has been sequenced (Fig. 3). The sequencing was performed according to the dideoxy method (22).

Together with the hybridization data of VI-3 with Ca 22-2 and Ca 22-1, it was possible to establish that the sequence of the VI-3 cDNA encompasses, at a minimum, 13 exons dispersed over a region of approximately 45 kb in the genomic DNA. Of these 13 exons, four are within the bcr region; one encompasses nucleotides 481-836 in the cDNA. The remaining exons are located 5' and 3' to the bcr region.

It is therefore possible to isolate a relatively small region of the VI-3 cDNA insert, e.g. 500 base pairs, and use this as a probe to detect both the $9q^+$ and $22q^-$ chromosomes in CML patients. A number of such probes can be constructed from this cDNA insert. Such probes will preferably be derived from the region of the VI-3 cDNA insert within the most 5' Bgl II restriction site and the most 5' Ava I restriction site (Fig. 2) and will detect all chromosomal abnormalities in the bcr region, including the breakpoint fragment of the K-562 cell line.

The VI-3 cDNA sequence is useful as a probe for detecting the Philadelphia translocation. This probe may be prepared as described in this specification or alternatively it or a substantially similar polynucleotide molecule may be prepared by any method of polynucleotide synthesis known to the art, e.g. enzymatic or chemical polynucleotide synthesis. Probes synthesized by this method can be used to probe cDNA libraries or genomic DNA for chromosome 22 DNA sequences. By using such probes, one of ordinary skill in the art can isolate chromosome 22 bcr sequences from any representative human cDNA library or from genomic DNA.

To exactly determine the location of the breakpoint in K-562, the DNA sequence of the breakpoint region of K-562 was compared with the normal genomic chromosome 22 DNA sequences. The two sequences are identical up to a point approximately in the center of the region shown in Fig. 5. 3' of this point, chromosome 9 sequences are joined to those of chromosome 22. No sequence homology is apparent between the chromosome 9 and 22 DNA sequences. Lack of homology between sequences involved in the 9;22 recombination event in CML is in concordance with the results obtained from sequence analysis of the 8;14 translocation in Burkitt lymphoma, in which no obvious homology was detected between the recombined sequence (13).

In addition, these sequence results demonstrate unambiguously that the breakpoint in K-562 has occurred within an intron of the bcr gene; DNA probes prepared from the VI-3 bcr CDNA were used in hybridization experiments to determine that the break is located in a region corresponding to the PstI/PvuII fragment in the VI-3 cDNA (see arrow Fig. 2); exons 5' of the breakpoint indicated in the figure remain on chromosome 22 in K-562. As in K-562, the chromosomal breakpoints in four other DNAs isolated from specimens of different patients have been located within introns of the bcr region.

The Philadelphia translocation therefore causes a break within a gene, of which the bcr is a part. In this translocation, chromosome 9 sequences including the c-abl oncogene are joined to the gene containing the bcr.

To examine the effect of the translocation of the human c-abl oncogene on its expression, polyA RNA was isolated from K-562 and control HELA cells. In concordance with results obtained by others (12), a c-abl specific probe detects a 6.0 and a 7.0 kb mRNA in both HELA cells and in K-562. In addition, a novel c-abl homologous mRNA of 8.5 kb is detected in K-562. Novel c-abl homologous mRNAs have also been detected in CML patient material (19, 20). Since the exact locations of the breakpoint in bcr is known for K-562, hybridization experiments were also performed in order to determine if the abnormal 8.5 kb mRNA found in CML cell line K562 was a bcr/c-abl mRNA as shown in Fig. 6. The experiments involved hybridization of the K-562 c-abl mRNA on blots as described. The hybridization analysis was performed using three different probes; a) a 0.6 kb EcoRI/BamHl fragment of genomic DNA from the 5' region of the c-abl gene containing an exon, b) a Pvu II/Pst I fragment of VI-3 cDNA 5' of the K-562 breakpoint (probe A, Fig. 2) and c) a Pst I/PvuII fragment of VI-3 cDNA, 3' of the K-562 breakpoint (probe B, Fig. 2). The c-abl probe detected the 8.5 kb amplified mRNA as in previous experiments. Probe A also detected the abnormal 8.5 kb mRNA, however probe B failed to detect this abnormal mRNA. These results conclusively show that in the Ph' positive cell line K-562 a bcr/c-abl mRNA is present. Moreover, we have detected an abnormally sized, 8.5 kb mRNA hybridizing to probe A in the RNAs isolated from bone marrow cells of 5 CML patients. This 8.5 kb mRNA appears analogous to the 8.5 kb mRNA in K-562 in that it hybridizes to probe A but not to probe B. Therefore, this mRNA is most probably characteristic of CML patients exhibiting the Philadelphia translocation. It follows that a bcr/c-abl protein product of this mRNA is also present and that it is highly probable that this protein is implicated in the causation and or progression of CML.

Probe A and B have also been used in Southern blot hybridization analysis of DNA derived from the K-562 and GM 637 cell lines. The probes hybridized to the expected restriction fragments. These results confirm that probe A picks up the abnormal restriction pattern associated with the Philadelphia translocation and is thus a good hybridization probe and useful in the diagnosis of CML.

Part of the VI-3 sequences described above does not seem to be present on a single-copy in the human genome. When a 5' probe is prepared from the VI-3 cDNA, encompassing all 5' sequences included in the 5' PvuII restriction enzyme fragment (see Fig. 2) and hybridized to total human DNA digested with different restriction enzymes, only fragments corresponding to the genomic DNA represented by the bcr region are seen under stringent washing conditions. Under less stringent washing conditions other sequences are also detected which hybridize to a 0.46 $Ps^+$ fragment in the VI-3 cDNA. These sequences may also be considered members of the bcr gene family, albeit less conserved. However, all probes prepared from VI-3 3' of the PvuII site detect multiple hybridizing fragments even after high stringency washings in human DNA. This indicates that at least part of the VI-3 cDNA is represented in a gene family. This gene family contains, at minimum, 4 members, as a single exon containing probe isolated from the 3' region of Ca 22-2 hybridizes to at least 4 discrete fragments in total human DNA cut with different restriction enzymes. Two members of this family have been molecularly cloned from the K-562 cosmid library described in this specification. Overlapping cosmids, spanning a region of 80 kb have been isolated, which contain a region of extremely close homology to the VI-3 3' sequences. These cosmids, of which the cosmid VKII-1 is the prototype, contain sequences also located on chromosome 22, but 5' (more toward the centromere) of the bcr region. The second member has been isolated in one cosmid spanning a region of 40 kb. This cosmid, VKII-3, also contains sequences hybridizing to the 0.46 kb $Ps^+$ fragment mentioned above. Of this gene family, at least three members are located on chromosome 22; as demonstrated by their presence in the DNA of a somatic cell hybrid, PG Me25-Nu (3). PG Me 25-Nu contains human chromosome 22 as its only human chromosome complement. The three members of this gene family on chromosome 22 are: a) the sequences represented by cosmid VKII-1 b) the sequences present in the *bcr* region and c) sequences 3' (telomeric) of the *bcr* region represented by the cosmid VKII-3, which are not amplified in K-562 and which are presumably translocated from chromosome 22 in the Ph'-translocation. Since the *bcr* region on chromosome 22 is specifically and consistently involved in the translocation resulting in the formation of the Ph'-chromosome, it is reasonable to assume that other members of its family may also be involved in specific translocations known to occur between chromosome 22 and other chromosomes, be it in neoplasia, such as in Ewing Sarcoma, or in hereditary disorders, such as the di-Georgio syndrome. The methods and probes of this invention, therefore may be useful in diagnosing other diseases associated with specific chromosomal translocations.

The largest cDNA, VI-3, containing an insert of 2.2 kb, was characterized in detail by restriction enzyme mapping (Fig. 4) and sequence analysis. The DNA sequence and translation are shown below. Amino acid residues are indicated with a one letter symbol below the sequence. The exact position of exons designated 1-6 (see Fig. 7A) within VI-3 *bcr* cDNA is shown by the numbers above the sequence. DNA sequencing was performed using the dideoxy termination method (22) on restriction fragments of the cDNA subcloned in M13 phage (36). All regions were sequenced on both strands.

The cDNA contains one long open reading frame starting at the polyG tail at the 5' end and continuing to nucleotide 1770 where a stopcodon is encountered. All other reading frames have numerous stopcodons within the entire region. The long open reading frame has the coding capacity for 589 amino acid residues corresponding to a protein of approximately 65,000 Mw. At the 3' end, a polyadenylation signal is found at nucleotide 2182 followed by a polyA tail beginning at base 2208, indicating that the cDNA contains the complete 3' end of the gene. Although translational start sequences are encountered at the 5' end, it is unlikely that this cDNA contains a complete copy of the mRNA as Northern blot hybridizations indicate the presence of bcr mRNAs with approximate sizes of 4.0 and 6.5 kb. Computer searches of newly isolated protein sequences derived directly from proteins or deduced from cDNA nucleotide sequences frequently result in the identification of proteins with partial homology. Such information is valuable, usually enabling the assignment of a preliminary function to an unknown protein. Therefore, the PIR FASTP program (32) was used to search for bcr homologous proteins: no proteins with significant homology were found, indicating that at present the bcr protein exhibits an unidentified cellular function

To determine the orientation of the bcr gene on chromosome 22, 5' and 3' probes were prepared from the VI-3 cDNA and hybridized to cosmids (5) containing human chromosome 22 sequences. This established that the 5' end of the bcr gene is toward the centromere of chromosome 22 and is retained after the Ph' translocation; the 3' end of the bcr gene points in the direction of the telomere and is translocated to chromosome 9 in the t(9;22). The cDNA hybridizes to restriction enzyme fragments distributed over a region of up to 45 kb of chromosome 22 DNA (Fig. 6A). Within this region, a minimum of 13 exons are present. To determine the exact position and number of exons within the breakpoint cluster region, all hybridizing regions in *bcr* were sequenced and compared with the VI-3 cDNA. As shown in Fig. 6B, four relatively small exons designated 1-4, are present within *bcr* and vary in size from 76-105 bp; in the cDNA, these exons correspond to nucleotides 483-836. Since *bcr* was defined as the area on chromosome 22 in which the Ph' breakpoints are found, it can be thus concluded that the breakpoints occur within a gene.

After determining the position of the exons within *bcr*, the question was addressed as to whether the breakpoints occur in exon or intron regions. For CML DNAs such as that of CML patient C481 this question is readily answered: Previously (5), we demonstrated a breakpoint within a 1.2 kb H/Bg *bcr* fragment in some of the CML DNAs such as that of patient C481. As no coding sequences are located within this region (see Fig. 6) patients such as C481 must have a chromosomal breakpoint in the intron between the exons designated 3 and 4.

A less simple situation was encountered in the DNAs of patients 0311068 and 7701C. Nonetheless, cloning of $9q^+$ breakpoint fragments from these DNAs and restriction enzyme analysis followed by Southern hybridization enabled us to locate the breakpoints between exons 2 and 3 (see Fig. 6). The breakpoints in the previously cloned (4,5) $9q^+$ breakpoint fragments of patients 0319129 and 02120185 were analyzed by DNA sequencing. In addition, we cloned the $22q^-$ breakpoint fragments of patient 0319129 and of the cell line K562 (28). DNA sequence analysis of the breakpoint regions of these fragments, in addition to that of the sequence of the corresponding chromosome 22 regions allowed us to define the point of translocation for these DNAs (see Fig. 6). None of the breakpoints analyzed here could be located within an exon, indicating that in the Ph' translocation breakpoints occur within intron regions of *bcr*. For 4 of the 6 DNAs analyzed, the translocation would result in the transcription of a mRNA that includes the *bcr* exons 1 and 2 (see Fig. 6), in two CML DNAs the third exon would be additionally incorporated in the *bcr*/c-*abl* mRNA. The third exon only encodes 25 amino acids, not substantially increasing the size of the polypeptide.

The DNA sequence of the junction point of the translocation might provide additional information, concerning the mechanism of chromosomal translocation. In Figure 8A, the sequence of the region containing the crossover point for translocation in the DNA of patient 0319129 is shown and compared with normal chromosome 22 and 9 DNA sequences. In 0319129 DNA, the chromosomal break has occurred in a rather "precise" manner, leading to the generation of a $22q^-$ and $9q^+$ sequence exactly reflecting the sequence of the normal chromosome 22 and 9 DNA sequences. However, at the breakpoint, a C nucleotide is found both in the $9q^+$ and $22q^-$ sequence, whereas the chromosome 22 sequence contains a G at that position. It is noteworthy, that both chromosomes 9 and 22 contain limited stretches of homology near the break (Fig. 8A underlined); a DNA search revealed homology of this region to human Alu-repetitive sequences.

Similar stretches of homologous sequences between chromosome 9 and 22 sequences are not apparent at the breakpoint in the DNA of patient 021015 (Fig. 8B) or that of K562 DNA. In the $9q^+$ DNA of patient 0212015, sequences are found between the breakpoints on chromosome 9 and 22 (see Fig. 8B) which are not present in the region sequenced of the normal chromosomes 9 and 22. This suggests that in the $9q^+$ chromosome a secondary event has taken place; it is possible that after translocation of chromosome 22 sequences to chromosome 9, a deletion affecting chromosome 9 sequences occurred. The arrow indicating the breakpoint on chromosome 9 (see Fig. 8B) would then represent the point of deletion. In addition, the chromosome 9 sequences of $9q^+$ fragment contain 13 nucleotide changes within a 81 bp stretch as compared to the normal chromosome 9 sequences. These changes may reflect differences between individuals (the control chromosome 9 sequences were isolated from a human lung carcinoma cosmid library); however, the number of nucleotide substitutions would be very high in such event. Moreover, the chromosome 22 part of the $9q^+$ fragment contains no nucleotide changes within either intron or exon sequences, favoring an explanation of inefficient DNA repair upon deletion of chromosome 9 sequences.

These results indicate, that the bcr is part of a gene oriented with its 5' end toward the centromere of chromosome 22. In the Philadelphia translocation, a break occurs within the bcr, and sequences from chromosome 9 containing the human c-abl oncogene in all Ph'-positive cases examined (5, 33), are translocated to the 3' of the truncated bcr gene. The joining of bcr and c-abl sequences is highly specific for CML as this configuration has been found in complex translocations (33) and even in the leukemic cells of one CML patient cytogenetically lacking the Ph' chromosome (34). Since the orientation of c-abl on chromosome 9 is also centromere 5' - 3' - telomere, bcr and c-abl are joined in a head-to-tail fashion on the Ph' chromosome. Although the distance from the most v-abl homologous exon to the physical breakpoint may vary from 14 kb (in the case of patient 0319129) to over 100 kb (in K562), the effect of the translocation on the expression of the bcr gene and c-abl seems to be very similar in different patients: in K562 and in Ph'-positive CML patients abnormal RNA transcripts of around 8.5 kb are detected, which hybridize both to c-abl and 5' bcr exon probes (28, 30). Definitive proof, that a hybrid mRNA exists, has been provided by the molecular cloning of a hybrid cDNA from K562 cells (31). The hybrid mRNAs must be the result of transcription initiating at the promoter of the bcr gene. Depending on the exact location of the breakpoint, the transcript will include all 5' exons in addition to either exons 1 and 2 or exons indicated 1, 2 and 3 of the bcr. Transcription continues into the c-abl oncogene, including, at minimum, the most 5' v-abl homologous exon and all exons 3' of it including the phosphotyrosine acceptor site (35). If the inclusion of exon 3 in the hybrid mRNA has any effect on the progression of the disease is as of yet unknown.

Chromosomal aberrations may be generated by specific events involving recombination - prone DNA sequences: alternatively, such recombination events could occur almost at random. In either case, a very limited number of translocations will result in gene alterations leading to the disruption of normal growth and differentiation. In the Ph' translocation, we have found that breakpoints on chromosome 9 are spread over a region of up to 100 kb. The breakpoints on chromosome 22 occur within a smaller region of around 5.0 kb. Nonetheless, no sequence homology can be found comparing breakpoint regions of different CML patients or coding regions of c-abl and bcr genes. Therefore the conclusion seems to be justified that the processes underlying the Ph' translocation are random recombination events. Once such recombinations result in a genomic configuration that enables the transcription of a hybrid bcr/abl mRNA, it can result in malignant proliferation of specific cell types. That such a hybrid mRNA is translated into protein is highly probable, as an abnormally sized 210,000 MW c-abl protein was detected in K-562 cells. In contrast to the normal c-abl protein, the P210 has tyrosine kinase activity (19).

## Materials and Methods

### Gel Electrophoresis and Hybridization

Restriction enzymes were purchased from either New England Biolabs or Bethesda Research Laboratories (BRL) and were used according to the suppliers' specifications. DNAs were digested with restriction enzymes, subjected to electrophoresis through 0.75% agarose gels, and transferred to nitrocellulose (Schleicher and Schuell, pH 7.9) essentially as described by Southern (25). Nick translation of probes and filter hybridization were as described (23, 24). Specific activity of the probes was 2-5 x $10^8$ cpm/ microgram. After hybridization, filters were exposed to XAR-2 film (Kodak) for up to 5 days at -70$^o$C with Dupont Lightning Plus intensifying screens.

### Preparation of DNA Probes

DNA probes were prepared by digestion of DNA with appropriate restriction enzymes, followed by electrophoresis through low-melting-point agarose (BRL). Desired bands were excised from gels and brought into solution by heating at 65$^o$C for 30 min. Agarose was removed by two extractions with phenol equilibrated with 0.3 M NaOAc, pH 5.0, and one extraction with phenol/chloroform/isoamyl alcohol (25:24:1). DNA was precipitated with ethanol and 0.2 M NaOAc, pH 4.8, in the presence of 20 micrograms/ml Dextran T-500 (Pharmacia) as carrier.

Northern Blot Hybridization

Northern blot analysis of c-*abl* mRNA in K-562 was conducted as follows: 20 micrograms of polyA RNA of K-562 and HELA cells was electrophoresed on a 1.2% agarose gel in the presence of formaldehyde (13). After blotting, nitrocellulose filters were hybridized to either the previously described 0.6 kb *Bam* HI/*Eco*RI probe representing the most 5' v-*abl* homologous exon of human c-*abl* (14, 15), or to probe A or B (see text and Fig. 2). Total RNA was isolated according to the LiCl/urea method (16). PolyA RNA was obtained after one passage of the RNA over oligo dT cellulose. Denatured, kinased λ-*Hind* III was used as a molecular weight standard.

Cosmid Cloning

Construction of the cosmid library, as well as procedures for the screening, isolation, and growth of recombinants, was as described (11, 25).

Cell Lines

The cell lines K-562 and GM 637 were obtained from the NIGMS, Human Genetic Mutants Cell Repository, Institute for Medical Research, Copewood and Davis Streets, Camden, New Jersey, 08103.

Analysis of CML Patients

Methods

Patients Studied

Five patients with Ph' positive CML have been studied. Four were found to have a normal translocation, one had a complex translocation. Patient 2128 is a 37 year old east Indian male, who was diagnosed to have CML on 6/17/82. He had a complex t(9;12), t(12;22) translocation (38). Chromosome studies revealed a 46, XY, Ph' karyotype. He is still in the chronic phase. His peripheral blood is normal.

Patient 2252 is a 45 year old white male. He presented with CML on 1/26/83 and had the standard t(9;22) translocation. His karyotype (46, XY, Ph') has remained unchanged. His peripheral blood is normal.

Patient 2397 is a 43 year old female with a 46, XX, t(9;22), Ph' karyotype. The first CML diagnosis was on 10/17/83. She has been evaluated regularly; her karyotype remains unchanged. Her peripheral blood is normal.

Patient 1708 is a white female presented with CML on 7/24/80. All 40 cells examined had the 46, XX, t(9;22), Ph' karyotype. The latest bone marrow aspirate was done on 7/9/84. In the 40 cells examined similar cytogenetic findings were found. She is in the chronic phase. For the last six years cytogenetic evaluation has been done serially. Peripheral blood cells stimulated with PHA are normal.

Patient 2172 is a 52 year old white male. He was originally diagnosed as having CML on 9/22/82. He has been followed since then for every three months. His karyotype remains 46, XY, t(9;22), Ph'. The PHA stimulated peripheral blood is normal.

Cytogenetic techniques

Initially, 20 metaphases were banded by G-banding each time a sample was drawn. Following this, 20 cells were banded by R-banding as described by Verma and Lubs (39). Metaphases were photographed in color, but printed in black and white. Cytogenetic evaluation was done by projecting the color slides.

RNA Isolation and Electrophoresis

Total RNA was isolated according to the LiCl/urea method (18). Fresh bone marrow samples (1-2.5 ml) were homogenized in 25 ml 3M LiCl, 6 M urea on ice for 2 x 30 seconds in a polytron homogenizer. The homogenate was kept 3 hours on ice. The precipitate was centrifuged at 10,000 g for 1 hour and dissolved in 3.2 ml of 1:1 mixture of buffer (10 mM Tris-HCl, pH 7.5, 5 mM EDTA, 0.2% SDS) and phenol: chloroform: isoamylalcohol (25:24:1). The waterphase was extracted two times with phenol/chloroform/ isoamylalcohol (25:24:1) and the RNA precipitated overnight at -20°C with 2.5 vol absolute ethanol and 011 vol. 3M NaOAc(pH 5.2). The RNA was rinsed three times in 70% ethanol and stored at -70°C. Poly A RNA was obtained after one passage of the RNA over oligo dT cellulose. 10 micrograms of Poly A RNA was electrophoresed on a 1% agarose gel in the presence of formaldehyde (16). Blotting to nitrocellulose (Schleicher and Schuell, pH 79) was essentially as described (16).

Hybridization

The $^{32}P$ labelled RNA probes are generated by transcription labeling according to the pSP riboprobe method (Promega Biotech). 1-5 x $10^8$ cpm $^{32}P$ labelled RNA was used per hybridization, corresponding to 0.5-2.5 x $10^8$ cpm/microgram input DNA. Filters were exposed to XAR-2 film (Kodak) at -70°C with Dupont Lighting plus intensifying screens.

Blots were first prehybridized in 100 ml 50% formamide, 50 mM sodium phosphate buffer pH 6.5, 5 x 5 SSC, 1 mM EDTA, 0.1% SDS, 2.5 x Denhardts, 250 micrograms/ml salmon sperm DNA, 250 micrograms/ml calfs liver RNA, 10 micrograms/ml poly A for four hours at 55°C. Hybridization was in 35 ml hybridization mix (4 parts prehybridization mix and 1 part 50% dextran sulphate) overnight at 55°C. The filters were washed 2 x 20 minutes in 20 mM sodium phosphate buffer, pH 6.5 1 mM EDTA, 0.1% SDS, 2.5 x SSC, at 55°C. Following this the filters were washed 2 x 20 minutes in 20 mM sodium phosphate buffer, pH 6.5, 1 mM EDTA, 0.1% SDS, 0.3 x SSC at 65°C, and similarly, in the same solution but with 0.1 and 0.03 x SSC consecutively, at 65°C.

Results

To demonstrate the presence of bcr/c-abl mRNAs in the bone marrow cells of CML patients a number of probes were required to perform the Northern blot analysis.

The majority of all Ph' breakpoints have been shown to occur in the intron sequences between the exons indicated as II and III or in the intron between the exons III and IV (see Fig. 9A) (5). This implies that the part of the cDNA corresponding to probe A (see Figure 9B) remains on chromosome 22 while the cDNA sequences corresponding to probe B are translocated to chromosome 9 in the Ph' translocation. Both probes were inserted in reverse orientation in pSP vectors enabling the isolation of $^{32}P$ labelled RNA complementary (anti-sense) to the normal bcr mRNA. Labelled probes were generated using this vector construct because previous experiments demonstrated that such probes result in an enhanced signal of at least five fold in Northern hybridizations compared with conventional nick-translated DNA probes. Similarly, a human c-abl fragment encompassing the most 5' v-abl homologous exon (probe C) was inserted into a pSP vector (17).

If a bcr/abl mRNA exists in the leukemic cells of CML patients we would expect to detect an abnormal sized abl transcript; the same abnormal sized mRNA should hybridize with the 5' bcr probe (probe A) but not with probe B, the 3' bcr probe as the genomic sequences corresponding to this probe are translocated to chromosome 9 in the t(9;22).

All patients have an 8.5 kb transcript that hybridizes both with the c-abl and the 5' specific bcr probe. The 3' specific bcr probe does not hybridize with this transcript, which excludes that it is a normal bcr messenger. The 8.5 kb messenger seems to be characteristic of Ph' positive cells, as it is not found in either normal control cells or cells of other types of leukemia (19,29). Three bands around 7.5, 7.0 and 4.5 kb are detected with both the 5' and the 3' bcr probe, indicating that these may represent the normal bcr mRNAs. The 7.5, 7.0 and 4.5 kb transcripts are also present in other human cell lines and tissues, including several other myeloid and lymphoid cell lines, erythroid precursor cells, skin, gut, kidney and spleen. None of the samples tested contained the 8.5 kb bcr related RNA.

The bone marrow cells from all the patients contain a 6.0 and 7.0 kb c-abl transcript. These transcripts are also present in normal cells (29) and represent the normal c-abl mRNAs. Presumably both are initiated at the same promoter but terminate differently at the 3' end of the abl oncogene. Although all five patients have both the 8.5 kb hybrid and the normal abl transcripts, the hybrid transcript is expressed at higher level.

## Discussion

Cells from patients with Ph' positive CML and different CML cell lines contain an abnormal c-abl related transcript of approximately 8.5 kb (19, 20, 29). We demonstrate that this abnormal mRNA is present in the RNAs of each five Ph' positive CML patients and that it represents a bcr/c-abl transcript.

The specific presence of this novel transcript in leukemic cells of CML patients supports once more the view that both c-abl and bcr are involved in CML and that the fusion of bcr c-abl sequences is a crucial event in the disease. The recent demonstration (40) of a breakpoint within bcr and the location of genomic bcr and c-abl sequences on chromosome 12 in a CML patient with a t(9;12) lacking a cytogenetically detectable Ph' chromosome provides further evidence.

In addition, this result emphasizes that cytogenetic detection of the Ph' chromosome has its limits as a prognostic and diagnostic marker. In approximately 15-25% of acute lymphoblastic leukemia (ALL) patients a Ph' chromosome is found in the leukemic cells. The inability to obtain high quality metaphase chromosome spreads from the leukemic cells of these patients has thus far hampered accurate diagnosis of this type of ALL. bcr DNA probes such as the ones used in this invention provide an alternative diagnostic tool for the detection of the Ph' translocation in Ph'- positive ALL and in some cytogenetically Ph' negative CML patients. Moreover, it is not unlikely, that in some ALL patients a hybrid transcript may also be found. Using v-abl probes, an abnormally sized c-abl RNA was detected in the human ALL cell line SMS-SB (41). Translation of a hybrid mRNA, as found in the five CML patients, would result in a bcr/abl fusion protein. Evidence for the existence of such protein has been found in the CMLO cell line K562, in which a similar bcr-c-abl transcript is found, an abnormally sized c-abl protein of 210 K molecular weight (P210) (14) is detected, suggesting that this protein is the translational product of the bcr/abl mRNA. Although the tumorigenic potential of this protein is unknown, it cannot be coincidence that it involved the c-abl oncogene since v-abl, the result of a recombination of murine leukemia virus and the mouse c-abl oncogene (4) induces a rapid B cell lymphoma in mice (43, 44).

How the bcr part of the abnormal bcr-c-abl hybrid protein could attribute to a possible tumorigenic activity is for the moment, unknown. However, studies with the CML cell line K562 may provide insight how the protein is altered: the abnormal P210 has tyrosine kinase activity in contrast to the normal P150 c-*abl* protein (14, 47). The *bcr* moiety of this molecule could therefore have altered the structure of the c-*abl* protein and so unmasked its associated tyrosine kinase activity.

REFERENCES:

1. J.D. Rowley, Nature 243, 290 (1973).

2. N. Heisterkamp et al., Nature 299, 747 (1982).

3. A. de Klein et al., Nature 300, 765 (1982).

4. N. Heisterkamp et al., Nature 306, 239 (1983).

5. J. Groffen et al., Cell 36, 93 (1984).

6. C.B. Lozzio and B.B. Lozzio, Blood 45, 321 (1975).

7. H.P. Koeffler and D.W. Golde, Blood 56, 344 (1980).

8. S.J. Collins and M.T. Groundine, Proc. Natl. Acad. Sci. U.S.A. 80, 4813 (1983).

9. J.R. Selden et al., Proc. Natl. Acad. Sci. U.S.A. 80, 7289 (1983).

10. J. Groffen et al., J. Exp. Med. 158, 9 (1983).

11. J. Groffen et al., Science 216, 1136 (1982).

12. S.J. Collins, I. Kubonishi, I. Miyoshi, M.T. Groudine, Science 225, 74 (1984).

13. J. Battey, et al., Cell 34, 799 (1983).

14. J.B. Konopka, S.M. Watanabe, O.N. Witte, Cell 37, 1035 (1984).

15. E. Southern, J. Mol. Biol. 98, 503 (1975).

16. T. Maniatis, E.F. Fritsch, J. Sambrook, Molecular Cloning (Cold Spring Harbor Laboratory, Cold Spring Harbor, N.Y. 1982), p. 202

17. N. Heisterkamp, J. Groffen, J.R. Stephenson, J. Mol. App. Genet. 2, 57 (1983).

18. C. Auffray and F. Rougeon, Eur. J. Biochem 107, 303 (1980).

19. E. Canaani et al., The Lancet 1984-I, 593 (1984).

20. S.J. Collins et al., Science, 225, 72 (1984)

21. H. Okayama and P. Berg, Mol. Cell. Biol. 3, 280 (1983).

22. Sanger et al, Proc Natl. Acad Sci USA 74, 5463 (1977).

23. Bernards R. Flavell R.A., Nucleic Acids Res 8: 1521-34 (1980).

24. Flavell R.A., Cell 15: 25-41 (1978).

25. Grosveld F.G. et al, Gene. 13 227 (1981).

26. Green, M.R., Maniatis, T. and Melton, D.A. Cell 32 681-694 (1983).

27. Melton, D.A., Krieg P, Green M.R. et al., in press Nucleic Acids Res (1984).

28. Grosveld, G. et al., submitted for publication.

29. Gale, R.P. and Cananni, E. Proc. Natl. Acad. Sci. USA 81, 5648 (1984).

30. Stam, K. et al., manuscript in preparation.

31. Cananni, E. et al., submitted for publication.

32. Lipman, D.F. and Pearson, W.R., Science, in press.

33. Bartram, C.R. et al. Nature 306, 277-280 (1983).

34. Bartram, C.R. et al., EMBO J., in press.

35. Groffen, J., Heisterkamp, N., Reynolds, F. Jr. and Stephenson, J.R. Nature 304, 167-169 (1983).

36. Mesing, J. and Viera, J. Gene 19, 269-276 (1982).

37. Groffen, J., Heisterkamp, N., Blennerhassett, G. and Stephenson, J.R. Virology 126, 213-227 (1983).

38. Chemitiganti, S., Verma, R.S., Silver, R.T., Coleman, M., and Dosik, H. Canc. Gen. and Cytogen. (1985); 14:61-65.

39. Verma, R.S. and Lubs, H.A., Am. J. Hum. Genet. (1975); 27:110-117.

40. Bartram C.R., Kleihauer E, de Klein A, Grosveld G, Teyssier J.R., Heisterkamp N., and Groffen J. EMBO J, in press.

41. Ozzanne, B., Wheeler, T., Zack, J., Smith, G. and Dale, B. Nature (1982); 299:744-747.

42. Wang, J.Y.J., Ledley, F., Goff, S., Lee, R., Groner, Y, and Baltimore, D. Cell (1984); 36:349-356.

43. Rosenberg N., Baltimore, D. and Scher, C.D. Proc Natl Acad Sci USA (1975); 72:1932-1936.

44. Abelson, H.T. and Rabstein, L.S. Cancer Res (1970); 30:2213-2222.

45. Ulrich, A., Bell, J.R., Chen, E.Y., Herrera, R., Petrusselli, M., Dull, T.J., Gray, A., Coussens, L., Liao, Y.C., Tsubokawa, M., Mason, A., Seeburg, P.H., Grunfeld, C., Rosen, O.M. and Ramachandran, J. Nature (1985); 313756-761.

46. Downward, J., Yarden, Y., Mayes, E., Scrace, G., Totty, N., Stockwell, P., Ullrich, A., Schlessinger, J, and Waterfield, M.D. Nature (1984); 307:521-525.

47. Kloetzer, W., Kurzrock, R., Smith, L., Talpaz, M., Spiller, M., Gutterman, J. and Arlinghaus, R. Virology (1985); 140:230-238.

What is claimed is:

1. A single-stranded nucleic acid molecule useful as a probe for detecting a chromosomal translocation having a nucleotide sequence which comprises at least one sequence present within an exon of a eucaryotic chromosomal gene and complementary to the mRNA encoded by the exon, the gene being characterized by the presence within it of at least one chromosomal breakpoint site into which another nucleotide sequence may be translocated.

2. A nucleic acid molecule which is complementary to the nucleic acid molecule of claim 1.

3. A DNA molecule of claim 1.

4. An RNA molecule of claim 1.

5. A nucleic acid molecule of claim 1 which comprises only nucleotide sequences present within exons.

6. A nucleic acid molecule of claim 1 which comprises nucleotide sequences present within exons and sequences present within introns.

7. A nucleic acid molecule of claim 6 which consists essentially of nucleotide sequences present within exons.

8. A nucleic acid molecule of claim 1 which comprises one sequence present within an exon located 3' of the chromosomal breakpoint sites.

9. A nucleic acid molecule of claim 1 which comprises more than one sequence present within exons wherein at least one exon sequence is located 3' of the chromosomal breakpoint sites.

10. A nucleic acid molecule of claim 1, wherein the molecule is from about 20 to about 8500 base pairs in length.

11. A nucleic acid molecule of claim 1, wherein the breakpoint site is located within an intron of the gene.

12. A nucleic acid molecule of claim 1, wherein the breakpoint site is located within an exon of the gene.

13. A nucleic acid molecule of claim 1, wherein the eucaryotic chromosomal gene is characterized by the presence within it of a breakpoint cluster region.

14. A nucleic acid molecule of claim 1, wherein the eucaryotic chromosome is a human chromosome.

15. A nucleic acid molecule of claim 1, wherein the chromosomal translocation is the Philadelphia translocation, wherein the eucaryotic chromosomal gene is characterized by the presence within it of a breakpoint cluster region and is located on human chromosome 22,wherein the exon is located 3' of a breakpoint site located within the cluster region and wherein the other nucleotide sequence is at least part of the c-abl oncogene.

16. A nucleic acid molecule of claim 15 which includes at least a portion of the sequence of the VI - 3 cDNA shown in Fig. 3.

17. A double-stranded DNA molecule which includes a single-stranded DNA molecule of claim 3.

18. A vector which comprises a DNA molecule of claim 3.

19. A plasmid of claim 18.

20. A cosmid of claim 18.

21. A phage of claim 18.

22. A nucleic acid molecule of claim 1 labelled with a detectable moiety.

23. A DNA molecule of claim 22.

24. An RNA molecule of claim 22.

25. A nucleic acid molecule of claim 22, wherein the moiety is radioactive.

26. A nucleic acid molecule of claim 15 labelled with a detectable moiety.

27. A DNA molecule of claim 26.

28. An RNA molecule of claim 26.

29. A method of detecting a chromosomal translocation on a eucaryotic chromosome which comprises cleaving the chromosomal DNA from the chromosome with an appropriate restriction enzyme or enzymes under suitable conditions so as to produce DNA fragments, treating the resulting fragments to obtain single-stranded DNA molecules, contacting the single-stranded DNA molecules so obtained with a single-stranded nucleic acid molecule of claim 22 under suitable conditions permitting hybridization of complementary single-stranded molecules, detecting the presence of hybridized molecules and thereby detecting the chromosomal translocation.

30. A method of detecting a chromosomal translocation which comprises cleaving chromosomal DNA with an appropriate restriction enzyme or enzymes under suitable conditions to produce DNA fragments, treating the resulting fragments by gel electrophoresis and denaturation to obtain single-stranded DNA molecules, recovering the single-stranded DNA molecules so obtained, immobilizing them on a suitable solid support, contacting the immobilized, single-stranded DNA molecules with a single-stranded nucleic acid molecule of claim 22 under suitable conditions permitting hybridization of complementary single-stranded molecules, detecting the presence of hybridized molecules and thereby detecting the chromosomal translocation.

31. A method of detecting the Philadelphia translocation in a human subject which comprises cleaving the subject's total chromosomal DNA with an appropriate restriction enzyme or enzymes under suitable conditions so as to produce DNA fragments, treating the resulting fragments to obtain single-stranded DNA molecules, contacting the single-stranded DNA molecules so obtained with a single-stranded nucleic acid molecule of claim 26 under suitable conditions permitting hybridization of complementary single-stranded molecules, detecting the presence of hybridized molecules and thereby detecting the Philadelphia translocation.

32. A method of detecting the Philadelphia translocation in a human subject which comprises cleaving the subject's total chromosomal DNA with an appropriate restriction enzyme or enzymes under suitable conditions so as to produce DNA fragments, treating the resulting fragments by gel electrophoresis and denaturation to obtain single-stranded DNA molecules, recovering the single-stranded DNA molecules so obtained, immobilizing them on a suitable solid support, contacting the immobilized, single-stranded DNA molecules with a single-stranded nucleic acid molecule of claim 26 under suitable conditions permitting hybridization of complementary single-stranded molecules, detecting the presence of hybridized molecules and thereby detecting the Philadelphia translocation.

33. A purified, hybrid RNA molecule useful as an indicator of a chromosomal translocation having a nucleotide sequence which comprises a sequence encoded by at least a portion of an exon of a eucaryotic chromosomal gene and a sequence encoded by at least a portion of another nucleotide sequence which has translocated into the gene.

34. An RNA molecule of claim 33, wherein the exon encoded sequence is located 5' of the sequence encoded by the translocated nucleotide sequence.

35. An RNA molecule of claim 34, wherein the translocated nucleotide sequence comprises at least part of an oncogene.

36. An RNA molecule of claim 35, wherein the chromosomal translocation is the Philadelphia translocation, wherein the eucaryotic chromosomal gene is chararacterized by the presence within it of a breakpoint cluster region and is located on human chromosome 22, wherein the exon is located 5' of the translocated nucleotide sequence and wherein the translocated nucleotide sequence comprises at least part of c-abl.

37. A purified, hybrid polypeptide useful as an indicator of a chromosomal translocation having an amino acid sequence which comprises a sequence encoded by at least a portion of an exon of a eucaryotic chromosomal gene and a sequence encoded by at least a portion of another nucleotide sequence which has translocated into the gene.

38. A polypeptide encoded by an RNA molecule of claim 35.

39. A polypeptide encoded by an RNA molecule of claim 36.

40. An antibody directed to an antigenic determinant unique to the polypeptide of claim 37.

41. A monoclonal antibody of claim 40.

42. An antibody of claim 40, wherein the antigenic determinant is present on the portion of the polypeptide encoded by exon nucleotide sequences located 5' of the translocated nucleotides.

43. An antibody of claim 40, wherein the antigenic determinant is present on the portion of the polypeptide encoded by the translocated nucleotide sequences.

44. An antibody directed to an antigenic determinant unique to the polypeptide of claim 38.

45. A monoclonal antibody of claim 44.

46. An antibody of claim 44, wherein the antigenic determinant is present on the portion of the polypeptide encoded by exon nucleotide sequences located 5' of the translocated oncogene.

47. An antibody of claim 44, wherein the antigenic determinant is present on the portion of the polypeptide encoded by nucleotide sequences of the translocated oncogene.

48. An antibody directed to an antigenic determinant unique to the polypeptide of claim 39.

49. A monoclonal antibody of claim 48.

50. An antibody of claim 48, wherein the antigenic determinant is present on the portion of the polypeptide encoded by exon nucleotide sequences located 5' of the translocated oncogene c-abl.

51. An antibody of claim 48, wherein the antigenic determinant is present on the portion of the polypeptide encoded by nucleotide sequences of the translocated oncogene c-abl.

52. An antibody of claim 40 labelled with a detectable moiety.

53. An antibody of claim 44 labelled with a detectable moiety.

54. An antibody of claim 48 labelled with a detectable moiety.

55. A single-stranded DNA molecule which is complementary to the RNA molecule of claim 33.

56. A DNA molecule of claim 55 labelled with a detectable moiety.

57. A method of detecting a chromosomal translocation by determining the presence of a polypeptide of claim 37 which comprises contacting a sample containing the polypeptide with an antibody directed to an antigenic site on the polypeptide, the antibody being labelled with a detectable moiety, under suitable conditions permitting the formation of an antibody-antigen complex, separating the unbound labelled antibody from the labelled antibody-antigen complex and detecting the presence of the labelled antibody-antigen complex.

58. A method of detecting a chromosomal translocation by determining the presence of a polypeptide of claim 38, which comprises contacting a sample containing the polypeptide with an antibody directed to an antigenic site on the polypeptide, the antibody being labelled with a detectable moiety, under suitable conditions permitting the formation of an antibody-antigen complex, separating the unbound labelled antibody from the labelled antibody-antigen complex and detecting the presence of the labelled antibody-antigen complex.

59. A method of detecting the Philadelphia translocation by determining the presence of the polypeptide of claim 39, which comprises contacting a sample containing the polypeptide with an antibody directed to an antigenic site on the polypeptide, the antibody being labelled with a detectable moiety, under suitable conditions permitting the formation of an antibody-antigen complex, separating the unbound labelled antibody from the labelled antibody-antigen complex and detecting the presence of the labelled antibody-antigen complex.

60. A method of detecting a chromosomal translocation by detecting the polypeptide of claim 37 which comprises contacting a sample containing the polypeptide with a measured amount of a first antibody directed to an antigenic site on the portion of the polypeptide encoded for by the exon nucleotide sequences of the gene into which another nucleotide sequence has translocated, first antibody being labelled with a detectable moiety, under suitable conditions so that an antibody-antigen complex is formed, contacting the complex so formed with a second antibody directed to an antigenic site on the portion of the polypeptide encoded for by the translocated nucleotide sequence, the second antibody being bound to a suitable solid support, under suitable conditions permitting the formation of a labelled antibody-antigen-antibody complex, separating the unbound labelled antibody from the labelled antibody-antigen-antibody complex bound to the solid support and measuring either the amount of the labelled antibody complex bound to the solid support or the amount of unreacted labelled antibody in order to determine the presence of the polypeptide.

61. A method of claim 60, wherein the first antibody is not labelled and is attached to a suitable solid support and where the second antibody is labelled with a detectable moiety.

62. A method of claim 60, wherein the antibodies are monoclonal antibodies.

63. A method of detecting a chromosomal translocation by detecting the polypeptide of claim 38 which comprises contacting a sample containing the polypeptide with a measured amount of a first antibody directed to an antigenic site on the portion of the polypeptide encoded for by the exon nucleotide sequences of the gene into which another nucleotide sequence has translocated, the first antibody being labelled with a detectable moiety, under suitable conditions so that an antibody-antigen complex is formed, contacting the complex so formed with a second antibody directed to an antigenic site on the portion of the polypeptide encoded for by the translocated nucleotide sequence, the second antibody being bound to a suitable solid support, under suitable conditions permitting the formation of a labelled antibody-antigen-antibody complex, separating the unbound labelled antibody from the labelled antibody-antigen-antibody complex bound to the solid support and measuring either the amount of labelled antibody complex bound to the solid support or the amount of unreacted labelled antibody in order to determine the presence of the polypeptide.

64. A method of claim 63, wherein the first antibody is not labelled and is attached to a suitable solid support and where the second antibody is labelled with a detectable moiety.

65. A method of claim 63, wherein the antibodies are monoclonal antibodies.

66. A method of detecting the Philadelphia translocation in a human subject by detecting the presence of the polypeptide of claim 39 which comprises contacting a sample obtained from the subject with a measured amount of first antibody directed to an antigenic site on the portion of the polypeptide encoded for the by <u>c-abl</u> oncogene, the first antibody being labelled with a detectable moiety, under suitable conditions permitting the formation of antibody-antigen complex, contacting the complex so formed with a second antibody directed to an antigenic site on the portion of the polypeptide encoded for by exon nucleotide sequences of the gene on chromosome 22, the second antibody being bound to a suitable solid support, under suitable conditions permitting the formation of a labelled antibody-antigen-antibody complex, separating the unbound labelled antibody from the labelled antibody-antigen-antibody complex bound to the solid support and measuring either the amount of labelled antibody-antigen-antibody complex bound to the solid support or the unbound labelled antibody in order to detect the presence of the polypeptide.

67. A method of claim 66, wherein the first antibody is not labelled and is bound to a solid support and where the second antibody is labelled.

68. A method of claim 66, wherein the antibodies are monoclonal antibodies.

69. A method of detecting a chromosomal translocation on a given eucaryotic chromosome which comprises isolating polyA mRNA molecules encoded by genes on the chromosome, separating the polyA mRNA molecules and immobilizing them on a suitable solid support, contacting the immobilized RNA molecules with an RNA molecule of claim 24 under suitable conditions permitting hybridization of complementary molecules, detecting the presence of hybridized molecules and thereby detecting the chromosomal translocation.

70. A method of detecting the Philadelphia translocation in a human subject which comprises isolating a human subject's polyA mRNA molecules, separating the mRNA molecules so obtained by gel electrophoresis, immobilizing the separated mRNA molecules on a suitable solid support, contacting the immobilized RNA molecules with an RNA molecule of claim 28 under suitable conditions permitting hybridization of complementary single-stranded molecules, detecting the presence hybridized molecules and thereby detecting abnormalities in the subject's mRNA caused by the Philadelphia translocation.

71. A method of detecting a chromosomal translocation which comprises isolating the total mRNA from the eucaryotic cell, immobilizing such molecules on a solid support, contacting the immobilized molecules with a single-stranded DNA molecule of claim 26 under conditions permitting hybridization of complementary single-stranded molecules, detecting the presence of hybridized molecule and thereby detecting the chromosomal translocation.

72. A method of diagnosing a disease by detecting the presence of a polypeptide of claim 37 associated with the disease.

73. A method of diagnosing chronic myelocytic leukemia, acute myelocytic leukemia or acute lymphocytic leukemia in humans by detecting the presence of the Philadelphia translocation.

74. A method of diagnosing chronic myelocytic leukemia, acute myelocytic leukemia or acute lymphocytic leukemia by detecting the presence of the Philadelphia translocation using the method of claim 31.

75. A method of diagnosing chronic myeloyctic leukemia, acute myelocytic leukemia or acute lymphocytic leukemia by detecting the presence of the Philadelphia translocation using the method of claim 59.

76. A method of diagnosing chronic myelocytic leukemia, acute myelocytic leukemia or acute lymphocytic leukemia by detecting the presence of the Philadelphia translocation using the method of claim 70.

0181635

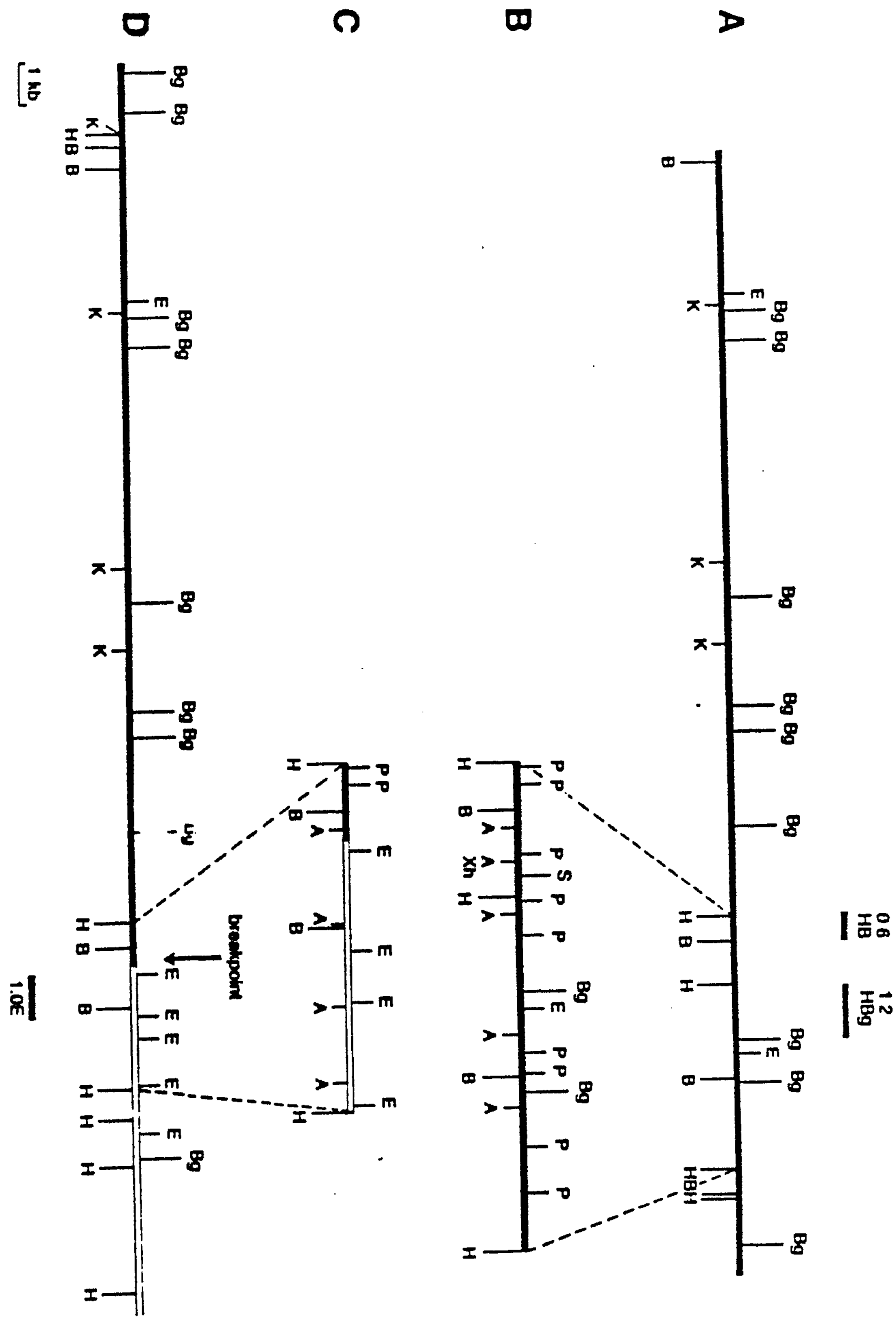


Fig. 1

0181635

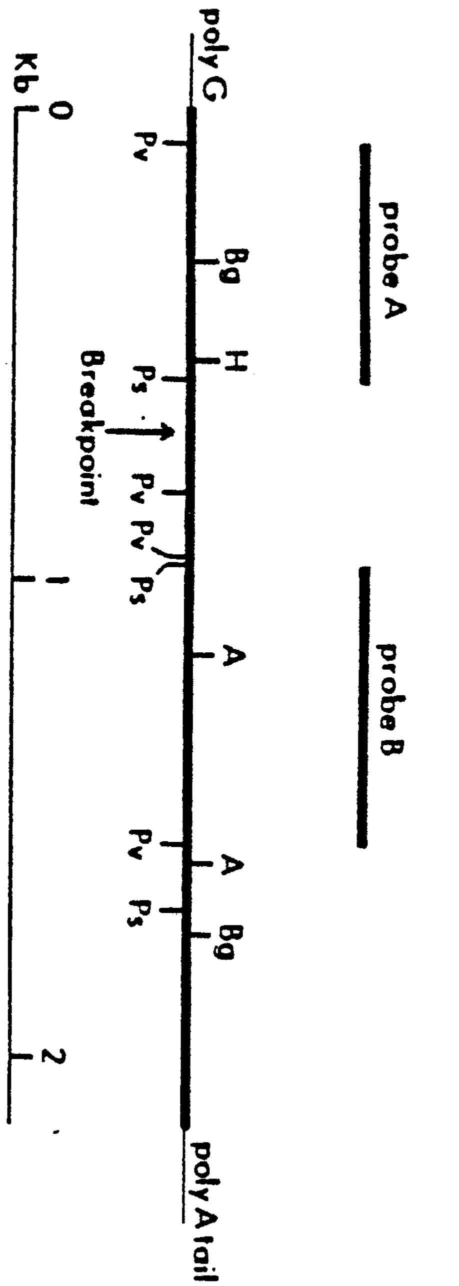


FIG. 2

Fig. 3A

```
          10          20          30          40          50
AG ACC TTC CTG TCC AGC ATC AAT GAG GAG ATC ACA CCC CGA CGG CAG TCC ATG ACG GTG
    N   F   L   S   S   I   N   E   E   I   T   P   R   R   Q   S   T   T   V

60          70          80          90          100         110
AAG AAG GGA GAG CAC CGG CAG CTG CTG AAG GAC AGC TTC ATG GTG GAG CTG GTG GAG GGG
 K   K   G   E   H   R   Q   L   L   K   D   S   F   M   V   E   L   V   E   G

120         130         140         150         160         170
GCC CGC AAG CTG CGC CAC GTC TTC CTG TTC ACC GAG CTG CTT CTC TGC ACC AGG CTC AAG
 A   R   K   L   R   H   V   F   L   F   T   E   L   L   L   C   T   K   L   K

180         190         200         210         220         230
AAG CAG AGC GGA GGC AAA ACG CAG CAG TAT GAC TGC AAA TGG TAC ATT CCG CTC ACG GAT
 K   Q   S   G   G   K   T   Q   Q   Y   D   C   K   W   Y   I   P   L   T   D

240         250         260         270         280         290
CTC AGC TTC CAG ATG GTG GAT GAA CTG GAG GCA GTG CCC AAC ATC CCC CTG GTG CCC GAT
 L   S   F   Q   M   V   D   E   L   E   A   V   P   N   I   P   L   V   P   D

300         310         320         330         340         350
GAG GAG CTG GAC GCT TTG AAG ATC AAG ATC TCC CAG ATC AAG AGT GAC ATC CAG AGA GAG
 E   E   L   D   A   L   K   I   K   I   S   Q   I   K   S   D   I   Q   R   E

360         370         380         390         400         410
AAG AGG GCG AAC AAG GGC AGC AAG GCT ACG GAG AGG CTG AAG AAG AAG CTG TCG GAG CAG
 K   R   A   N   K   G   S   K   A   T   E   R   L   K   K   K   L   S   E   Q

420         430         440         450         460         470
GAG TCA CTG CTG CTG CTT ATG TCT CCC AGC ATG GCC TTC AGG GTG CAC AGC CGC AAC GGC
 E   S   L   L   L   L   M   S   P   S   M   A   F   R   V   H   S   R   N   G

      1
480         490         500         510         520         530
AAG AGT TAC ACG TTC CTG ATC TCC TCT GAC TAT GAG CGT GCA GAG TGG AGG GAG AAC ATC
 K   S   Y   T   F   L   T   S   S   D   Y   E   R   A   E   W   R   E   N   I

                       1_______2
540         550         560         570         580         590
CGG GAG CAG CAG AAG AAG TGT TTC AGA AGC TTC TCC CTG ACA TCC GTG GAG CTG CAG ATG
 R   E   Q   Q   K   K   C   F   R   S   F   S   L   T   S   V   E   L   Q   M

600         610         620         630         640         650
CTG ACC AAC TCG TGT GTG AAA CTC CAG ACT GTC CAC AGC ATT CCG CTG ACC ATC AAT AAG
 L   T   N   S   C   V   K   L   Q   T   V   H   S   I   P   L   T   I   N   K
```

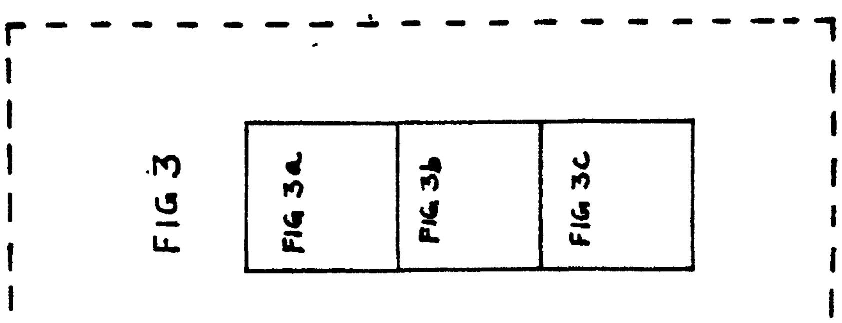

Fig. 3B

```
 2—3
660         670         680         690         700         710
GAA GAT GAT GAG TCT CCG GGG CTC TAT GGG TTT CTG AAT GTC ATC GTC CAC TCA GCC ACT
 E   D   D   E   S   P   G   L   Y   G   F   L   N   V   I   V   H   S   A   T

                  3————4
720         730         740         750         760         770
GGA TTT AAG CAG AGT TCA AAT CTG TAC TGC ACC CTG GAG GTG GAT TCC TTT GGG TAT TTT
 G   F   K   Q   S   S   N   L   Y   C   T   L   E   V   D   S   F   G   Y   F

                                                                    4———5
780         790         800         810         820         830
GTG AAT AAA GCA AAG ACG CGC GTC TAC AGG GAC ACA GCT GAG CCA AAC TGG AAC GAG GAA
 V   N   K   A   K   T   R   V   Y   R   D   T   A   E   P   N   W   N   E   E

840         850         860         870         880         890
TTT GAG ATA GAG CTG GAG GGC TCC CAG ACC CTG AGG ATA CTG TGC TAT GAA AGG TGT TAC
 F   E   I   E   L   E   G   S   Q   T   L   K   I   L   C   Y   E   K   C   Y

900         910         920         930         940         950
AAC AAG ACG AAG ATC CCC AAG GAG GAC GGC GAG AGC AGC GAC AGA CTC ATG GGG AAG GGC
 N   K   T   R   I   P   K   E   D   G   E   S   T   D   R   L   M   G   K   G

        5————6
960         970         980         990         1000        1010
CAG GTC CAG CTG GAC CCG CAG GCC CTG CAG GAC AGA GAC TGG CAG CGC ACC GTC ATC GCC
 Q   V   Q   L   D   P   Q   A   L   Q   D   R   D   W   Q   R   T   V   I   A

      6——
1020        1030        1040        1050        1060        1070
ATG AAT GGG ATC GAA GTA AAG CTC TCG GTC AAG TTC AAC AGC AGG GAG TTC AGC TTG AAG
 M   N   G   I   E   V   K   L   S   V   K   F   N   S   R   E   F   S   L   K

1080        1090        1100        1110        1120        1130
AGG ATG CCG TCC CGA AAA CAG ACA GGG GTC TTC GGA GTC AAG ATT GCT GTG GTC ACC AAG
 R   M   P   S   R   K   Q   T   G   V   F   G   V   K   I   A   V   V   T   K

1140        1150        1160        1170        1180        1190
AGA GAG AGG TCC AAG GTG CCC TAC ATC GTG CGC CAG TGC GTG GAG GAG ATC GAG CGC CGA
 T   K   R   E   R   S   K   V   P   Y   I   V   R   Q   C   V   E   E   I   E

1200        1210        1220        1230        1240        1250
GGC ATG GAG GAG GTG GGC ATC TAC CGC GTG TCC GGT GTG GCC ACG GAC ATC CAG GCA CTG
 G   M   E   E   V   G   I   Y   R   V   S   G   V   A   T   D   I   Q   A   L

1260        1270        1280        1290        1300        1310
AAG GCA GCC TTC GAC GTC AAT AAC AAG GAT GTG TCG GTG ATG ATG AGC GAG ATG GAC GTG
 K   A   A   F   D   V   N   N   K   D   V   S   V   M   M   S   E   M   D   V

1320        1330        1340        1350        1360        1370
AAC GCC ATC GCA GGC ACG CTG AAG CTG TAC TTC CGT GAG CTG CCC GAG CCC CTC TTC ACT
 N   A   I   A   G   T   L   K   L   Y   F   R   E   L   P   E   P   L   F   T

1380        1390        1400        1410        1420        1430
GAC GAG TTC TAC CCC AAC TTC GCA GAG GGC ATC GCT CTT TCA GAC CCG GTT GCA AAG GAG
 D   E   F   Y   P   N   F   A   E   G   I   A   L   S   D   P   V   A   K   E

1440        1450        1460        1470        1480        1490
AGC TGC ATG CTC AAC CTG CTG CTG TCC CTG CCG GAG GCC AAC CTG CTC ACC TCC CTT TTC
 S   C   M   L   N   L   L   L   S   L   P   E   A   N   L   L   T   F   L   F
```

Fig. 3C

1500 1510 1520 1530 1540 1550
CTT CTG GAC CAC CTG AAA AGG GTG GCA GAG AAG GAG GCA GTC AAT AAG ATG TCC CTG CAC
L L D H L K K V A E K E A V N K M S L H

1560 1570 1580 1590 1600 1610
AAC CTC GCC ACG GTC TTT GGC CCC ACG CTG CTC CGG CCC TCC GAG AAG GAG AGC AAG CTC
N L A T V F G P T L L R P S E K E S K L

1620 1630 1640 1650 1660 1670
CCT GCC ACC CCC AGC CAG CCT ATC ACC ATG ACT GAC AGC TGG TCC TTG GAG GTC ATG TCC
P A N P S Q P I T M T D S W S L E V M S

1680 1690 1700 1710 1720 1730
CAG GTC CAG GTG CTG CTG TAC TTC CTG CAG CTG GAG GCC ATC CCT GCC CCG GAC AGC AAG
Q V Q V L L Y F L Q L E A I P A P D S K

1740 1750 1760 1770 1780 1790
AGA CAG AGC ATC CTG TTC TTC ACC GAA GTC TAA AGG TCC CAG TCC ATC TCC TGG AGG CAG
R Q S I L F S T E V -

1800 1810 1820 1830 1840 1850
ACA GAT GGC CTG GAA ACC TCT GGC TAA TCG GGC CAT CCG TAG AGC AGG AAC CTT CCT GAG

1860 1870 1880 1890 1900 1910
GTG TCC TTG GGC CAC CCC CAA GTG TTG GGC CAT CTG CCA AGA GAC AGC GAC CCA AAG CCG

1920 1930 1940 1950 1960 1970
AAG GAC AGG TGG CCT GGG CAG ATC TCG CCC AGG TCT GGG AGC CCC AGG CTG GCC TCA GAC

1980 1990 2000 2010 2020 2030
TGT GGT TTT TTA TGT GGC CAC CCG AGG GCG CCC CAA GCC AGT TCA TCT.CAG AGT CCA GGC

2040 2050 2060 2070 2080 2090
CTG ACC CTG GGA GAC AGG GTG AAG GGA GTG ATT TTT ATG AAC TTA ACT TAG AGT CTA AAA

2100 2110 2120 2130 2140 2150
GAT TTC TAC TGG ATC ACT TGT CAA GAT GCG CCC TCT CTG GGG AGA AGG GAA CGT GAC CGG

2160 2170 2180 2190 2200 2210
ATT CCC TCA CTG TTG TAT CTT GAA TAA ACG CTG CTG CTT CAT CCT GTG AAA AAA AAA AAA

2220 2230 2240 2250
AAA AAA AAA AAA AAA AAA AAA AAA AAA AAA AAA AAA

Fig. 4

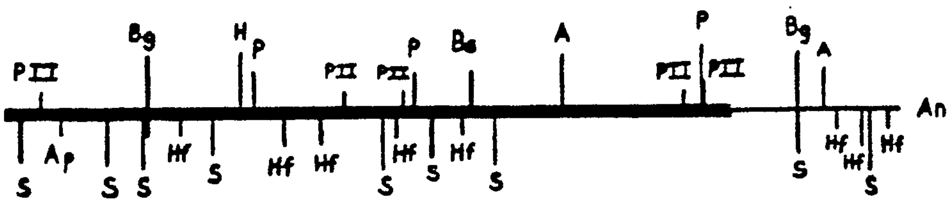
PII
Bg
H
P
PII
PII
P
Bs
A
PII
P
PII
Bg
A
An
S
Ap
S
S
Hf
S
Hf
Hf
S
Hf
S
Hf
S
S
Hf
Hf
S
Hf

0181635

FIG. 5

| | |
|---|---|
| chromosome 22 | CTGGCCGCTGTGGAGTGTTTGTGCTGGTTGATGC |
| $22q^-$ in K562 | CTGGCCGCTGTGGAGTGGGTTTTATCAGCTTCCA |
| | chromosome 22 — chromosome 9 |

FIG. 6

# HYBRID mRNA IN CML

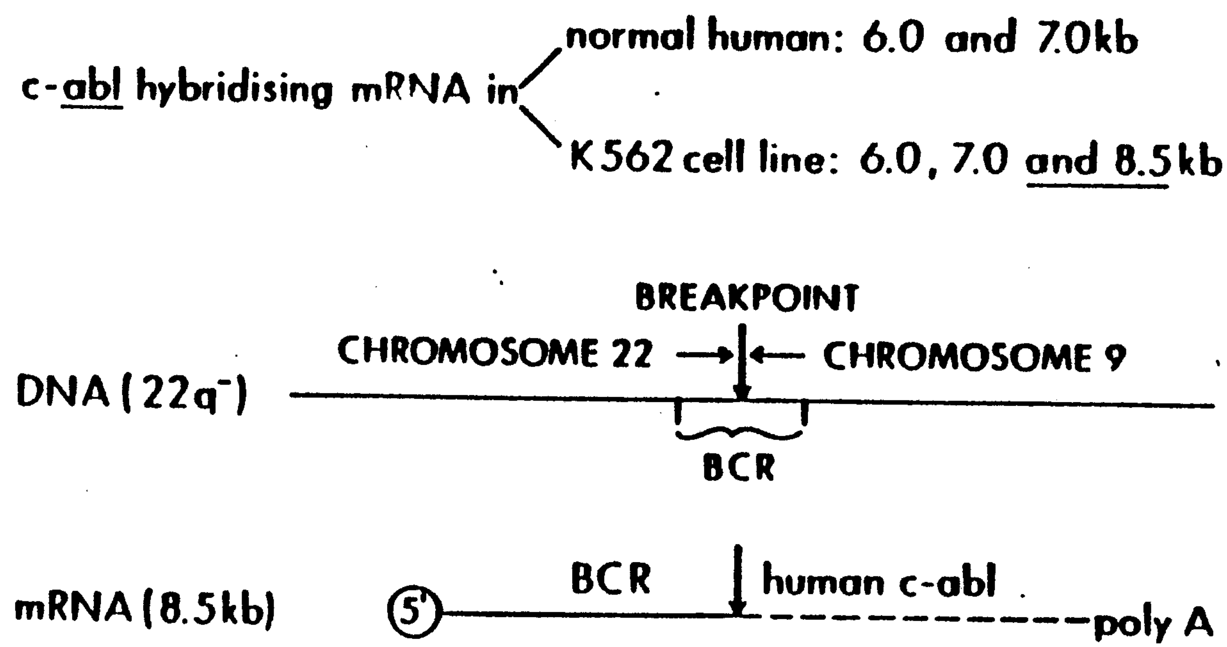

0181635

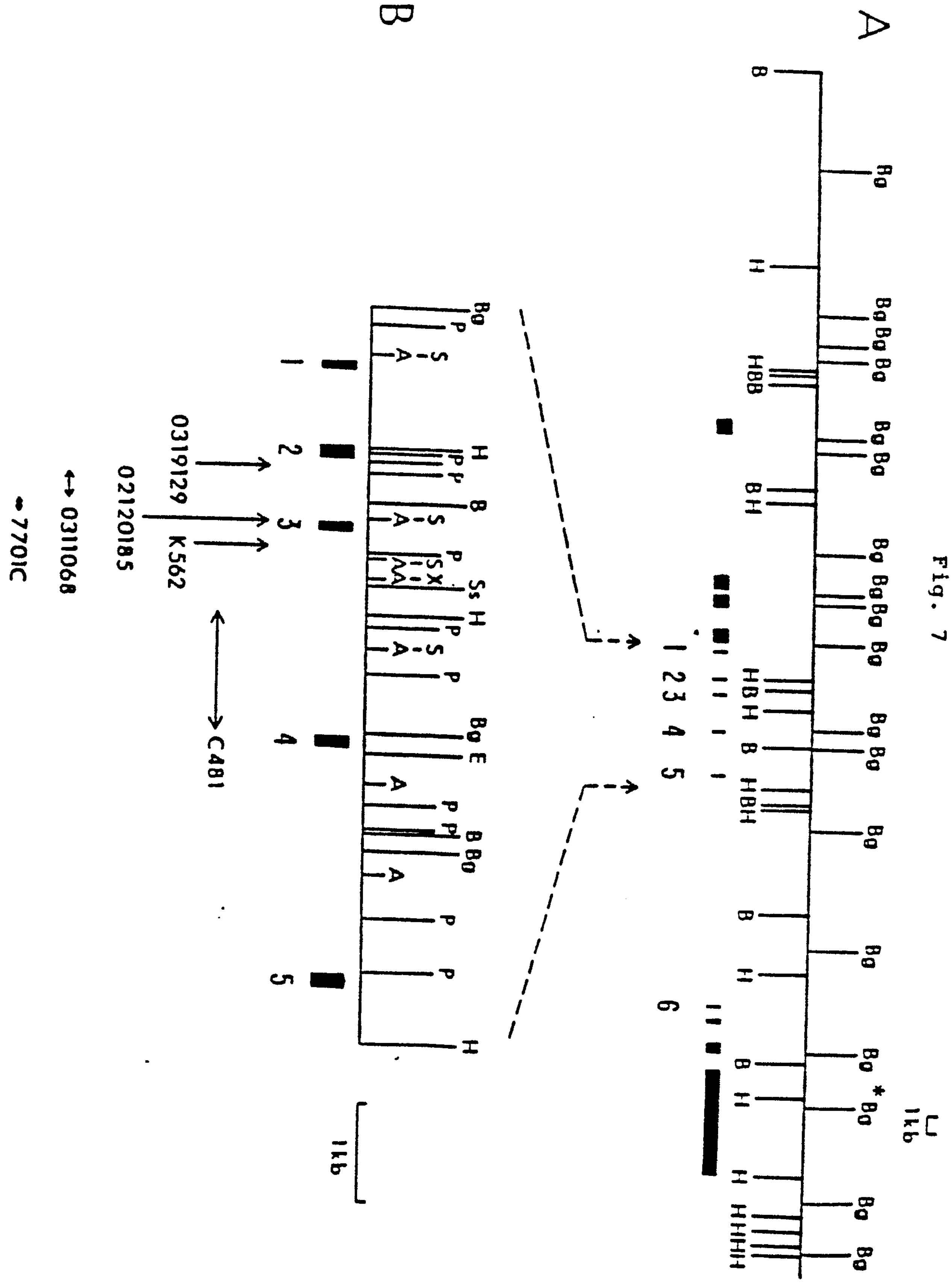


Fig. 7

Fig. 8

A

0319129

```
9     TTGTTGCTTTTGGAGATAGGGTCTTGCTCTGTCACCCAGGCTGGACTGCAGTGGCACAATCACAGCTCAC

9q+   TTGTTGCTTTTGCAGATAGGGTCTTGCTCTGTCACCCAGGCTTAGGAGCAGTTTCTCCCTGAGTGGCTGC
22q-  GTTAGGGCCTCTTGTCTCCTCCCAGGAGTGGACAAGGTGGCTGGACTGCAGTGGCACAATCACAGCTCAC

22    GTTAGGGCCTCTTGTCTCCTCCCAGGAGTGGACAAGGTGGGTTAGGAGCAGTTTCTCCCTGAGTGGCTGC
```

B

02120185

```
9     CCCGGGTTCAAGCGATTCTCCTGCCTCAGCCTCTTGAGTAGCTGGAACTACAGGCACGTGCCACCATGCC
9q+   CCCGGGTTCACGTGATACTCTTGCCTCAGCTTCCTGCATAGCTGGGACTACAGGCACCCACCACCACGCC
22    TGACACTGGCTTACCTTGTGCCAGGCAGATGGCAGCCACACAGTGTCCACCGGATGGTTGATTTTGAAGC

9     CAGCTAATTTTTTTGTTTGTTTGTTTGTTTGTTTTCCTGAGACGGAGTCTCACTGTCACCCAGGCTGGAG
9q+   CAGCTAATTTTGTGTATGTTTAGTAGAGACGAGGTTTCACAGAAGCTGACCTCTTTGGTCTCTTGCGCAG
22    AGAGTTAGCTTGTCACCTGCCTTCCCTTTCCCGGGACAACAGAAGCTGACCTCTTTGATCTCTTGCGCAG

9     TGCAGTGGCGTAATCTTGGCTCACTGCAACCTCCACCTCCCGGG
9q+   ATGATGAGTCTCCGGGGCTCTATGGGTTTCTGAATGTCATCGTC
22    ATGATGAGTCTCCGGGGCTCTATGGGTTTCTGAATGTCATCGTC
      exon 3
```

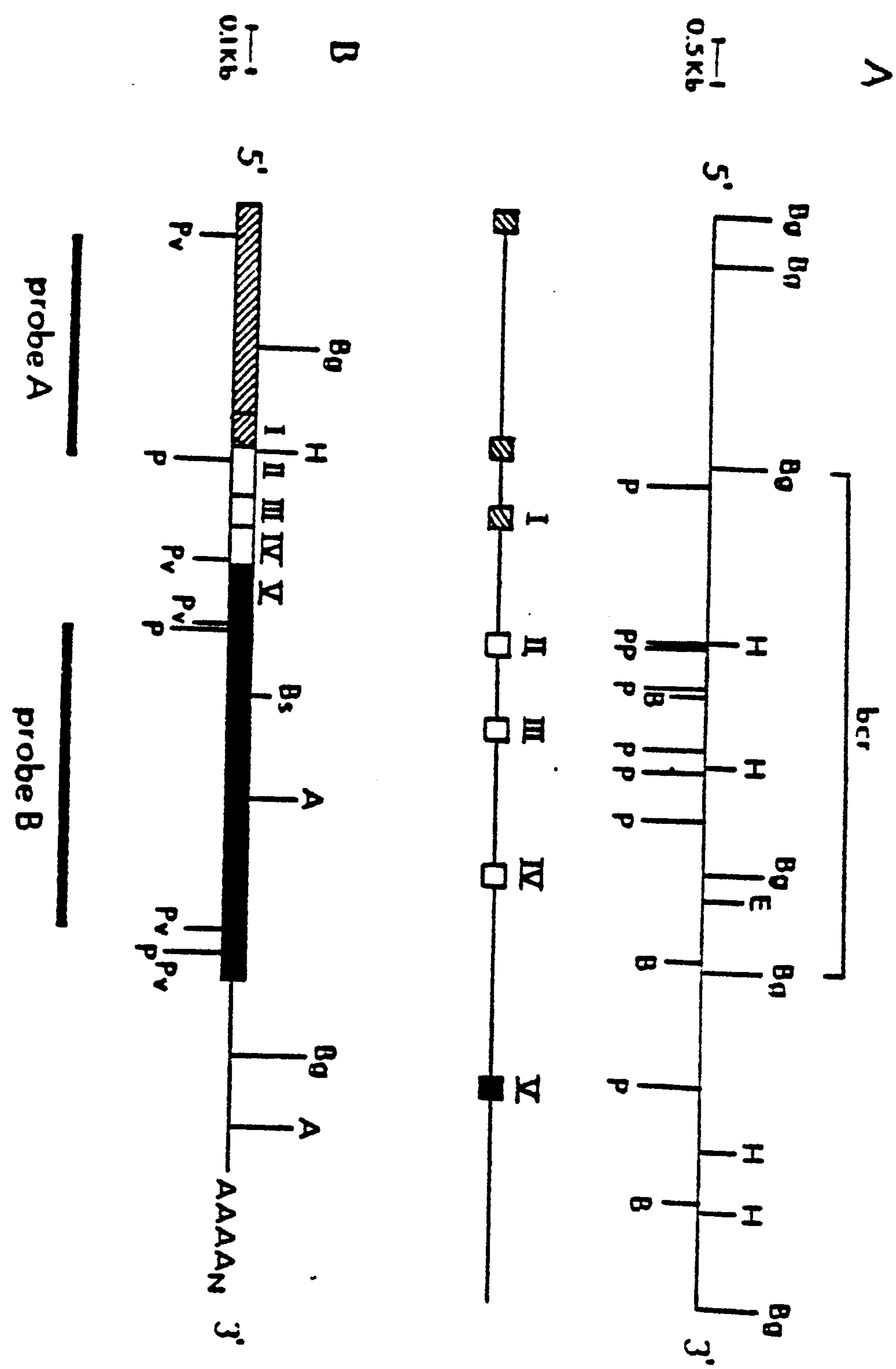
A
0.5Kb
5'
3'
Bg
P
H
B
E
bcr
I
II
III
IV
V
B
0.1kb
Pv
H
Bs
A
AAAAN
probe A
probe B

FIG. 9